(19) Europäisches Patentamt
European Patent Office
Office européen des brevets

(11) **EP 3 557 222 B1**

(12) **EUROPEAN PATENT SPECIFICATION**

(45) Date of publication and mention of the grant of the patent:
**02.03.2022 Bulletin 2022/09**

(21) Application number: **18887197.4**

(22) Date of filing: **09.07.2018**

(51) International Patent Classification (IPC):
*G16H 50/30* (2018.01)    *G16H 50/50* (2018.01)
*G01N 15/06* (2006.01)    *G06F 17/11* (2006.01)

(52) Cooperative Patent Classification (CPC):
**G16H 50/30; G01N 15/0656; G06F 17/11; G16H 50/50**

(86) International application number:
**PCT/CN2018/095014**

(87) International publication number:
**WO 2019/128184 (04.07.2019 Gazette 2019/27)**

(54) **CALCULATION METHOD AND TESTING SYSTEM FOR CUMULATIVE DUST EXPOSURE TO RESPIRABLE DUST OF MINE WORKERS**

BERECHNUNGSVERFAHREN UND PRÜFSYSTEM FÜR KUMULATIVE STAUBEXPOSITION GEGENÜBER EINATEMBAREM STAUB VON MINENARBEITERN

PROCÉDÉ DE CALCUL ET SYSTÈME D'ANALYSE D'EXPOSITION À LA POUSSIÈRE CUMULATIVE DE POUSSIÈRE RESPIRABLE DE TRAVAILLEURS MINIERS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.12.2017 CN 201711429874**

(43) Date of publication of application:
**23.10.2019 Bulletin 2019/43**

(73) Proprietor: **CHONGQING RESEARCH INSTITUTE CO., LTD. OF CHINA COAL TECHNOLOGY & ENGINEERING GROUP Jiulongpo District Chongqing 400039 (CN)**

(72) Inventors:
 • **LI, Dewen**
   **Chongqing 400037 (CN)**
 • **WANG, Jie**
   **Chongqing 400030 (CN)**
 • **ZHAO, Zheng**
   **Chongqing 400037 (CN)**
 • **WU, Fuxiang**
   **Chongqing 400042 (CN)**
 • **LIU, Guoqing**
   **Chongqing 400037 (CN)**
 • **XU, Kui**
   **Chongqing 400039 (CN)**

 • **GUO, Shengjun**
   **Chongqing 400037 (CN)**
 • **SUI, Jinjun**
   **Chongqing 400037 (CN)**
 • **HUI, Lifeng**
   **Chongqing 400037 (CN)**
 • **ZHANG, Qiang**
   **Chongqing 400037 (CN)**
 • **JIAO, Min**
   **Chongqing 401254 (CN)**
 • **DENG, Qin**
   **Chongqing 400037 (CN)**
 • **YAN, Dan**
   **Chongqing 400037 (CN)**
 • **ZHENG, Lei**
   **Chongqing 400037 (CN)**
 • **LIU, Haichen**
   **Chongqing 400038 (CN)**
 • **WANG, Junting**
   **Wulanchabu Inner Mongolia 013764 (CN)**
 • **WU, Liang**
   **Chongqing 402781 (CN)**

**(Cont. next page)**

(74) Representative: **Lermer, Christoph**
**LermerRaible Patent- u. Rechtsanwalts**
**PartGmbB**
**Lessingstrasse 6**
**80336 München (DE)**

(56) References cited:
**CN-A- 104 500 142**  **CN-A- 105 089 703**
**CN-A- 105 089 703**  **CN-A- 107 358 363**
**CN-A- 108 225 996**  **JP-A- 2001 272 391**
**US-A1- 2008 122 634**

• **ZHOU GANG ET AL: "The diffusion behavior law of respirable dust at fully mechanized caving face in coal mine: CFD numerical simulation and engineering application", PROCESS SAFETY AND ENVIRONMENTAL PROTECTION, INSTITUTION OF CHEMICAL ENGINEERS, RUGBY, GB, vol. 106, 19 December 2016 (2016-12-19), pages 117-128, XP029924711, ISSN: 0957-5820, DOI: 10.1016/J.PSEP.2016.12.005**

## Description

### Field of the Invention

The present invention relates to a calculation method for a dust exposure amount of respiration dust, and in particular to a calculation method and a measuring system for a cumulative dust exposure amount of respiration dust of a mine operator.

### Background of the Invention

Respiration dust, i.e., respirable-sized dust, is part of the dust generated in a mine operation process. Dust with a particle size less than 7.07 $\mu$m is generally considered as the respirable-sized dust, which can be inhaled into lungs by humans and deposited in alveolar regions, generating great harm to the health of the mine operators. The workers with long-term inhalation of the respirable dust would suffer lung diseases, resulting in the pneumoconiosis. The pneumoconiosis cannot be cured at present, but it can be prevented. The main measure to prevent the pneumoconiosis is to supervise a cumulative dust exposure amount of respiration dust of the operators.

However, due to the large number of mine employees in China, it is difficult to supervise occupational hazards. At present, the modes of self-inspection and spot check are mainly used. The authenticity of self-inspection monitoring is poor, and the spot check monitoring has the deficiencies of poor timeliness, narrow coverage and human factor influence. The individual cumulative dust exposure amount of the respiration dust monitoring cannot be carried out by the two modes in combination with the individual differences of the operators, early warning of the occupational hazards of the operators cannot be achieved, and countermeasures cannot be taken in time. With the development of the Internet and the big data technology, it has become an irresistible trend to establish an early warning information database for occupational hazards in mines, build a third-party support platform for occupational hazards based on cloud computing and big data, and realize early warning. Document CN 105 089 703 A discloses a method for calculating a cumulative dust exposure amount of respiration dust of a mine operator, comprising the steps of: obtaining a wind flow distribution law of the operating surface on-site and data output by a respiration dust concentration sensor; providing a calculation model based on an occupational hazards computation model in combination with the respiration dust concentration and space/time data of an operator; and calculating the cumulative dust exposure amount of respiration dust of the mine operator using a value TWA dependent on time and concentration, real-time positioning and position structure of the mine operator, total lung ventilation of a general adult divided by rise/day; and exposure times of respiration of local dust concentrations.

### Summary of the Invention

The objects of the present invention are to at least solve the technical problems existing in the prior art, and the present invention innovatively proposes a P computer-implemented calculation method and a measuring system for a cumulative dust exposure amount of respiration dust of a mine operator.

In order to achieve the above objects of the present invention, according to a first aspect of the present invention, the present invention provides a method for calculating a cumulative dust exposure amount of respiration dust of a mine operator, including: obtaining a distribution law of respiration dust concentration of an operating surface based on a wind flow distribution law of the operating surface on-site and data output by a respiration dust concentration sensor arranged on the operating surface.

The calculation method further includes: creating a calculation model for an individual dust exposure amount of respiration dust based on a multilevel accumulation mathematical model in combination with the distribution law of respiration dust concentration of the operating surface and a personnel space-time trajectory of mine operation, and calculating the cumulative dust exposure amount of respiration dust of the mine operator by using the calculation model for the individual dust exposure amount of respiration dust, wherein the calculation model for the individual dust exposure amount of respiration dust is as follows:

$$D = \sum_{j=0}^{n} Q_{kj} C_{kj} t_{kj}$$

wherein D is the individual dust exposure amount of respiration dust,

k is the serial number of the operator on the operating surface, and $k \geq 1$,
n is the number of position points of the operator in the personnel space-time trajectory on the operating surface, j is the sequence number of the position point in the personnel space-time trajectory, and $1 \leq j \leq n$,

$t_{kj}$ is the stay time of the k-th operator at the j-th point position and is obtained based on the individual space-time trajectory,

$Q_{kj}$ is an individual respiratory flow of the k-th operator at the j-th point position, and

$C_{kj}$ is an average respiration dust concentration value of the k-th operator at the j-th point position within a time $\triangle t$ and is obtained based on the distribution law of the respiration dust concentration,

wherein the process of obtaining the distribution law of respiration dust concentration of the operating surface comprises the following steps:

S1: establishing a two-phase flow mathematical model of the wind flow and the respiration dust and a physical model of the operating surface; wherein the two-phase flow mathematical model of the wind flow and the respiration dust comprises:

gas phase momentum equations:

$$\frac{\partial}{\partial t}(\rho_g v_{gx}) + \frac{\partial}{\partial x}(\rho_g v_{gx} v_{gx}) + \frac{\partial}{\partial y}(\rho_g v_{gy} v_{gx}) + \frac{\partial}{\partial z}(\rho_g v_{gz} v_{gx}) = -\frac{\partial p_g}{\partial x} + \frac{\partial}{\partial x}\tau_{gxx} + \frac{\partial}{\partial y}\tau_{gyx} + \frac{\partial}{\partial z}\tau_{gzx} + \rho_g F_{gx} + \sum_K F_{gpKx} + v_{gx}S$$

$$\frac{\partial}{\partial t}(\rho_g v_{gy}) + \frac{\partial}{\partial x}(\rho_g v_{gx} v_{gy}) + \frac{\partial}{\partial y}(\rho_g v_{gy} v_{gy}) + \frac{\partial}{\partial z}(\rho_g v_{gz} v_{gy}) = -\frac{\partial p_g}{\partial y} + \frac{\partial}{\partial x}\tau_{gxy} + \frac{\partial}{\partial y}\tau_{gyy} + \frac{\partial}{\partial z}\tau_{gzy} + \rho_g F_{gy} + \sum_K F_{gpKy} + v_{gy}S$$

$$\frac{\partial}{\partial t}(\rho_g v_{gz}) + \frac{\partial}{\partial x}(\rho_g v_{gx} v_{gz}) + \frac{\partial}{\partial y}(\rho_g v_{gy} v_{gz}) + \frac{\partial}{\partial z}(\rho_g v_{gz} v_{gz}) = -\frac{\partial p_g}{\partial z} + \frac{\partial}{\partial x}\tau_{gxz} + \frac{\partial}{\partial y}\tau_{gyz} + \frac{\partial}{\partial z}\tau_{gzz} + \rho_g F_{gz} + \sum_K F_{gpKz} + v_{gz}S$$

particle phase momentum equations:

$$\frac{\partial}{\partial t}(\rho_{pK} v_{pKx}) + \frac{\partial}{\partial x}(\rho_{pK} v_{gx} v_{pKx}) + \frac{\partial}{\partial y}(\rho_{pK} v_{gy} v_{pKx}) + \frac{\partial}{\partial z}(\rho_{pK} v_{gz} v_{pKx}) = -\frac{\partial p_{pK}}{\partial x} + \frac{\partial}{\partial x}\tau_{pKxx} + \frac{\partial}{\partial y}\tau_{pKyx} + \frac{\partial}{\partial z}\tau_{pKzx} + \rho_{pK} F_{pKx} + J_{pKx} + v_{gx}S_{pK}$$

$$\frac{\partial}{\partial t}(\rho_{pK} v_{pKy}) + \frac{\partial}{\partial x}(\rho_{pK} v_{gx} v_{pKy}) + \frac{\partial}{\partial y}(\rho_{pK} v_{gy} v_{pKy}) + \frac{\partial}{\partial z}(\rho_{pK} v_{gz} v_{pKy}) = -\frac{\partial p_{pK}}{\partial y} + \frac{\partial}{\partial x}\tau_{pKxy} + \frac{\partial}{\partial y}\tau_{pKyy} + \frac{\partial}{\partial z}\tau_{pKzy} + \rho_{pK} F_{pKy} + J_{pKy} + v_{gy}S_{pK}$$

$$\frac{\partial}{\partial t}(\rho_{pK} v_{pKz}) + \frac{\partial}{\partial x}(\rho_{pK} v_{gx} v_{pKz}) + \frac{\partial}{\partial y}(\rho_{pK} v_{gy} v_{pKz}) + \frac{\partial}{\partial z}(\rho_{pK} v_{gz} v_{pKz}) = -\frac{\partial p_{pK}}{\partial z} + \frac{\partial}{\partial x}\tau_{pKxz} + \frac{\partial}{\partial y}\tau_{pKyz} + \frac{\partial}{\partial z}\tau_{pKzz} + \rho_{pK} F_{pKz} + J_{pKz} + v_{gz}S_{pK}$$

gas phase energy equations:

$$\frac{\partial}{\partial t}(\rho_g c_{pg} T_g) + \frac{\partial}{\partial x}(\rho_g v_{gx} c_{pg} T_g) - \frac{\partial p_g}{\partial t} = -\frac{\partial}{\partial x}(\lambda_g \frac{\partial T_g}{\partial x}) + S c_{pg} T_g - \sum_K n_{pK} Q_K - Q_{rg}$$

$$\frac{\partial}{\partial t}(\rho_g c_{pg} T_g) + \frac{\partial}{\partial y}(\rho_g v_{gy} c_{pg} T_g) - \frac{\partial p_g}{\partial t} = -\frac{\partial}{\partial y}(\lambda_g \frac{\partial T_g}{\partial y}) + S c_{pg} T_g - \sum_K n_{pK} Q_K - Q_{rg}$$

$$\frac{\partial}{\partial t}(\rho_g c_{pg} T_g) + \frac{\partial}{\partial z}(\rho_g v_{gz} c_{pg} T_g) - \frac{\partial p_g}{\partial t} = -\frac{\partial}{\partial z}(\lambda_g \frac{\partial T_g}{\partial z}) + S c_{pg} T_g - \sum_K n_{pK} Q_K - Q_{rg}$$

and

particle phase energy equations:

$$\frac{\partial}{\partial t}(\rho_{pK} c_{pK} T_{pK}) + \frac{\partial}{\partial x}(\rho_{pK} v_x c_{pK} T_{pK}) = \frac{\partial}{\partial x}(\lambda_{pK} \frac{\partial T_{pK}}{\partial x}) + S_{pK} c_{pg} T_g + n_{pK}(Q_K - Q_{rK})$$

$$\frac{\partial}{\partial t}(\rho_{pK} c_{pK} T_{pK}) + \frac{\partial}{\partial y}(\rho_{pK} v_y c_{pK} T_{pK}) = \frac{\partial}{\partial y}(\lambda_{pK} \frac{\partial T_{pK}}{\partial y}) + S_{pK} c_{pg} T_g + n_{pK}(Q_K - Q_{rK})$$

$$\frac{\partial}{\partial t}(\rho_{pK} c_{pK} T_{pK}) + \frac{\partial}{\partial z}(\rho_{pK} v_z c_{pK} T_{pK}) = \frac{\partial}{\partial z}(\lambda_{pK} \frac{\partial T_{pK}}{\partial z}) + S_{pK} c_{pg} T_g + n_{pK}(Q_K - Q_{rK})$$

wherein $\rho$ is the fluid density in kg/m$^3$, $v$ is the fluid velocity in m/s, $p$ is the fluid pressure in Pa, $t$ is the time in s, S is the source term in kg/m$^3$, $\tau$ is the shear force in N/m$^3$, F is the volume force in N/m$^3$, $c_p$ is the constant pressure specific heat in W*s/kg*K, $\lambda$ is the heat conductivity coefficient in W/m*K, n is the number of particles, $Q_K$ is the heat taken away by the K-th phase particles from the fluid due to heat convection and its unit is W*s/m, $Q_r$ is the radiant heat in W*s/m, $J_{pK}$ is the component force of the diffusion flow of the K-th phase particles and its unit is N/m$^3$, $F_{gpK}$ is the resultant force of a particle phase acting on a fluid phase and its unit is N/m$^3$, and wherein the subscripts in the parameters mean that: g represents the gas phase, p represents the particle phase and K represents the K-th phase particles;

S2: performing numerical simulation based on the two-phase flow mathematical model of the wind flow and the respiration dust and the physical model of the operating surface, to obtain a numerical simulation result of the distribution law of respiration dust concentration;

S3: obtaining a distribution characteristic of respiration dust concentration of the operating surface based on data of respiration dust concentration provided by the respiration dust concentration sensor disposed on the operating surface;

S4: obtaining wind speed data based on the wind flow distribution law of the operating surface; and

S5: verifying and correcting the numerical simulation result of the distribution law of respiration dust concentration by using the distribution characteristic of respiration dust concentration of the operating surface and the wind speed data, and obtaining the distribution law of respiration dust concentration of the operating surface.

A real-time and online continuous obtaining method for the dust exposure amounts of the respiration dust of the operators in underground coal dust workplaces was solved, which lays a foundation for the early warning of the pneumoconiosis of the mine operators and for the archives management of the cumulative dust exposure amounts of the operators for enterprises.

The numerical simulation result is verified and corrected based on an onsite wind speed and the actual distribution characteristic of respiration dust concentration, so that the distribution law of respiration dust concentration of the operating surface can truly reflect the condition of the operating surface, which lays an accurate data basis for the establishment of the calculation model for the individual dust exposure amount.

The mathematical model has high precision and accuracy.

In still another preferred embodiment of the present invention, the method for obtaining the wind flow distribution law of the operating surface on-site is as follows: obtaining the wind flow distribution law of the operating surface based on wind speed data and position information provided by wind speed sensors disposed on the operating surface, and wherein

the number of the wind speed sensors is plural, which are evenly or unevenly disposed on a windward side and a leeward side of the operating surface, at a wind flow interference site and within an operating range of the operator.

The wind flow distribution law provides an on-site and real-time wind speed data basis for the acquisition of the distribution law of respiration dust concentration of the operating surface.

In still another preferred embodiment of the present invention, the number of the respiration dust concentration sensors is plural, which are evenly or unevenly disposed in an area with relatively even dust diffusion on the operating surface, at fixed operating places and within the operating range.

The measurement points of the respiration dust concentration are reasonably arranged, so that the distribution law of respiration dust concentration of the operating surface can reflect the actual situation as accurately and completely as possible, improving the calculation accuracy of the calculation model for the individual dust exposure amount of respiration dust.

In still another preferred embodiment of the present invention, the respiration dust concentration sensor is periodically calibrated, and the calibration process includes:

a standardizing step: standardizing an individual respiration dust detection device by using an individual respiration dust sampler;

a connecting step: carrying, by the operator, the standardized individual respiration dust detection device to the position where the respiration dust concentration sensor is located, and establishing data communication with the respiration dust concentration sensor; and

a calibration step: comparing an onsite detected value of the individual respiration dust detection device with that of the respiration dust concentration sensor, and calibrating the respiration dust concentration sensor based on the detected value of the individual respiration dust detection device.

Measuring errors, caused by harsh environmental factors such as high humidity on-site, of the respiration dust concentration sensor are corrected, so that the detected value of the respiration dust concentration sensor can truly reflect the respiration dust concentration on-site.

In still another preferred embodiment of the present invention, the personnel space-time trajectory is obtained based on the position of the operator reported by a personnel positioning system and the stay time at the positions; and wherein

the personnel positioning system includes a plurality of positioning receivers carried by the operator and a device point for receiving information sent by the positioning receivers, a topological relationship between the device point and the positioning receivers is a tree structure, and topological relationships among the positioning receivers include points and arc segments, arc segments and arc segments, and arc segments and points.

Based on the tree structure, hierarchical traversal is performed, and a confidence interval of the positioning information is obtained according to the real-time state information of the two receivers, so that the positioning data of the personnel are more accurate and the errors brought by the personnel positioning system can be well controlled.

In still another preferred embodiment of the present invention, the method further includes a step of performing onsite correction on the calculation model for the individual dust exposure amount of respiration dust, and the step includes:

using a single or multiple individual respiration dust detection devices to continuously detect at the position where the respiration dust concentration sensor is arranged on the operating surface for a time ti, and correcting the calculation model for the individual dust exposure amount of respiration dust by using the detected value.

The error of the calculation model for the individual dust exposure amount of respiration dust, which is caused by the deviation of the distribution law of respiration dust concentration, is mainly eliminated, which makes the calculated value of the calculation model closer to an actual value of the dust exposure amount.

In still another preferred embodiment of the present invention, the method further includes a step of optimizing the calculation model for the individual dust exposure amount of respiration dust, and the step includes:

respectively carrying, by a plurality of operators, the individual respiration dust detection devices to operate for at least one work shift on the operating surface, and optimizing the calculation model for the individual dust exposure amount of respiration dust by using the detected values of the plurality of individual respiration dust detection devices.

The error of the calculation model for the individual dust exposure amount of respiration dust, which is caused by the deviation of the individual time trajectory, is mainly optimized, so that the error of the calculation model is within a reasonable range, and the individual dust exposure amount of the operator can be truly reflected.

In order to achieve the above objects of the present invention, according to a second aspect of the present invention, the present invention provides a system for measuring a cumulative dust exposure amount of respiration dust of a mine operator using a method as described above, including a data processing center, respiration dust concentration sensors, wind speed sensors, humidity sensors, a personnel positioning system, a data memory and a calibration instrument; wherein the respiration dust concentration sensors, the wind speed sensors, the humidity sensors and the personnel positioning system are in wireless connection with the data processing center, and the data memory is in wired connection with the data processing center, and wherein

the storage content of the data memory includes a personnel space-time trajectory of all operators of each operating surface of a mine and a distribution law of respiration dust concentration of the operating surface;

the number of the respiration dust concentration sensors is plural, which are evenly or unevenly disposed in an area with relatively even dust diffusion on the operating surface, at fixed operating places and within an operating range;

the number of the wind speed sensors is plural, which are evenly or unevenly disposed on a windward side and a leeward side of the operating surface, at a wind flow interference site and within the operating range of the operator.

the number of humidity sensors is plural, which are evenly or unevenly disposed on the operating surface;

the personnel positioning system includes a plurality of positioning receivers carried by the operator and a device point for receiving information sent by the positioning receivers, a topological relationship between the device point and the positioning receivers is a tree structure, and topological relationships among the positioning receivers include points and arc segments, arc segments and arc segments, and arc segments and points;

the calibration instrument includes an individual respiration dust detection device and an individual respiration dust sampler; and

the data processing center obtains the distribution law of respiration dust concentration and the personnel space-time trajectory respectively based on the data output by the respiration dust concentration sensors, the wind speed sensors, the personnel positioning system and the humidity sensors, and combines the calculation model for the individual dust exposure amount of respiration dust to calculate the cumulative dust exposure amount of respiration dust of the operator.

The system can calculate the cumulative dust exposure amount of each operator by using the calculation model for the individual dust exposure amount of respiration dust depending on the wind speed, the respiration dust concentration, the humidity information and the personnel space-time trajectory measured in real-time, and can predict the cumulative dust exposure amount of each operator, which may be taken as the basis for the management of occupational hazards of enterprises, achieving effective supervision of the respiration dust hazards of the operators of the enterprises.

## Brief Description of the Drawings

Fig.1 is a flow diagram of a calculation method for a cumulative dust exposure amount of respiration dust of a mine operator in a specific embodiment of the present invention.

Fig.2 is a detailed flow diagram of a distribution law of respiration dust concentration in a specific embodiment of the present invention.

Fig.3 is a detailed flow diagram of a calibration process of a respiration dust concentration sensor in a specific embodiment of the present invention.

Fig.4 is a schematic diagram of a tree structure of a personnel positioning system in a specific embodiment of the present invention.

Fig.5 is a system block diagram of a measuring system for a cumulative dust exposure amount of respiration dust of a mine operator in a specific embodiment of the present invention.

Fig.6 is a schematic diagram of unit-set multiple-point correction of the calculation model for the individual dust exposure amount of respiration dust in a specific embodiment of the present invention.

Fig.7 is a schematic diagram of multi-set multi-point correction of a calculation model for an individual dust exposure amount of respiration dust in a specific embodiment of the present invention.

## Detailed Description of the Embodiments

The embodiments of the present invention are described in detail below, and the examples of the embodiments are illustrated in the drawings, wherein the consistently identical or similar reference signs refer to the identical or similar elements or elements having identical or similar functions. The embodiments described below with reference to the drawings are exemplified, which are merely used for explaining the present invention and cannot be construed as limiting the present invention.

In the description of the present invention, it should understood that orientation or position relationships indicated by terms "longitudinal", "transverse", "upper", "lower", "front", "back", "left", "right", "vertical", "horizontal", "top", "bottom", "inside", "outside" and the like are orientation or position relationships shown in the drawings, are merely used for the convenience of describing the present invention and simplifying the description, rather than indicating or implying that the devices or components referred to have specific orientations and constructed and operated in particular orientations, and thus cannot be constructed as limitations to the present invention.

In the description of the present invention, unless otherwise specified and limited, it should be noted that the terms "installation", "connected" and "connection" should be understood broadly, and can be, for example, mechanical connection or electrical connection, can be the internal communication of two elements, can be directly connected and can also be indirectly connected through an intermediate medium, and those skilled in the art can understand the specific meanings of the above terms according to specific conditions.

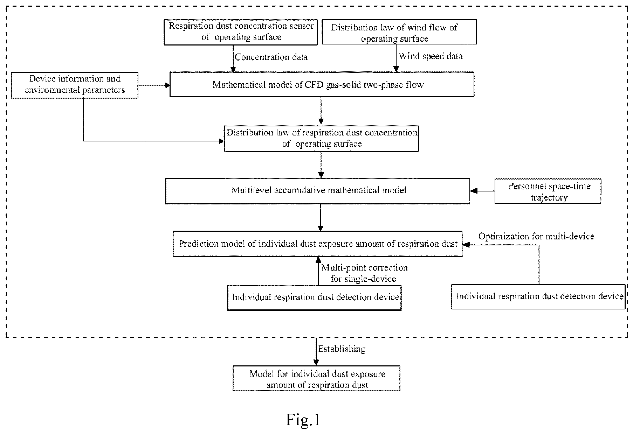

The present invention provides a method for calculating a cumulative dust exposure amount of respiration dust of a mine operator. Fig.1 is a flow diagram of the calculation method for the cumulative dust exposure amount of respiration dust of the mine operator in a specific embodiment of the present invention, including:

obtaining a distribution law of respiration dust concentration of an operating surface based on a wind flow distribution law of the operating surface on-site and data output by a respiration dust concentration sensor arranged on the operating surface;

creating a calculation model for an individual dust exposure amount of respiration dust based on a multilevel accumulation mathematical model in combination with the distribution law of respiration dust concentration of the operating surface and a personnel space-time trajectory of mine operations, and calculating the cumulative dust exposure amount of respiration dust of the mine operator by using the calculation model for the individual dust exposure amount of respiration dust, wherein the calculation model for the individual dust exposure amount of respiration dust is as follows:

$$D = \sum_{j=0}^{n} Q_{kj} C_{kj} t_{kj}$$

wherein D is the individual dust exposure amount of respiration dust; k is the serial number of the operator on the operating surface, and $k \geq 1$; n is the number of position points of the operators in the personnel space-time trajectory on the operating surface, j is the sequence number of the position point in the personnel space-time trajectory, and $1 \leq j \leq n$; $t_{kj}$ is the stay time of the k-th operator at the j-th point position; $Q_{kj}$ is an individual respiratory flow of the k-th operator at the j-th point position; and $C_{kj}$ is an average respiration dust concentration value of the k-th operator at the j-th point position within a time $\Delta t$ and is obtained based on the distribution law of the respiration dust concentration. In the present embodiment, $\Delta t$ may be a positive integer multiple of $t_{kj}$, or is at least greater than the time length of $t_{kj}$. $Q_{kj}$ is obtained based on a measured value by performing a respiration flow measuring on the operators of all or key positions of a mine in advance, or is calculated based on a human respiration model by performing a respiration resistance measurement on all operators of the mine.

In the present embodiment, the multilevel accumulative mathematical model contains two or more layers of implicit parameter changes in the accumulation of variables, so modelling is performed on the implicit parameter changes to complete the modelling of overall parameters, and the derivation process of the mathematical model is as follows:

Considering the variables $x_j^{(0)}$ and $x_j^{(1)}$, j=1, 2...N. It can be known that:

$$j=1$$

$$k=1, \quad x_1^{(0)}(2) = \alpha_1 + \beta_1 \{-\frac{1}{2}[x_1^{(1)}(1) + x_1^{(1)}(2)]\} + e_{21},$$

$$k=2, \quad x_1^{(0)}(3) = \alpha_1 + \beta_1 \{-\frac{1}{2}[x_1^{(1)}(2) + x_1^{(1)}(3)]\} + e_{31},$$

$$\bullet \bullet \bullet$$

$$k=n, \quad x_1^{(0)}(n) = \alpha_1 + \beta_1 \{-\frac{1}{2}[x_1^{(1)}(n-1) + x_1^{(1)}(n)]\} + e_{n1},$$

j=2

k=1, $$x_2^{(0)}(2) = \alpha_2 + \beta_2\{-\frac{1}{2}[x_2^{(1)}(1) + x_2^{(1)}(2)]\} + e_{22}$$,

k=2, $$x_2^{(0)}(3) = \alpha_2 + \beta_2\{-\frac{1}{2}[x_2^{(1)}(2) + x_2^{(1)}(3)]\} + e_{32}$$,

• • •

k=n, $$x_2^{(0)}(n) = \alpha_2 + \beta_2\{-\frac{1}{2}[x_2^{(1)}(n-1) + x_2^{(1)}(n)]\} + e_{n2}$$,

j=N

k=1, $$x_N^{(0)}(2) = \alpha_N + \beta_N\{-\frac{1}{2}[x_N^{(1)}(1) + x_N^{(1)}(2)]\} + e_{2N}$$,

k=2, $$x_N^{(0)}(3) = \alpha_N + \beta_N\{-\frac{1}{2}[x_N^{(1)}(2) + x_N^{(1)}(3)]\} + e_{3N}$$,

• • •

k=n, $$x_N^{(0)}(n) = \alpha_N + \beta_N\{-\frac{1}{2}[x_N^{(1)}(n-1) + x_N^{(1)}(n)]\} + e_{nN}$$,

The above form can be written as a neutralization formula:

$$x_j^{(0)}(k) = \alpha_j + \beta_j\{-\frac{1}{2}[x_j^{(1)}(k-1) + x_j^{(1)}(k)]\} + e_{kj}$$

Assuming $y_{kj} = x_j^0(k)$, $x_{kj} = -\frac{1}{2}[x_j^{(1)}(k-1) + x_j^{(1)}(k)]$

Then the neutralization formula is converted into:

$$y_{kj} = \alpha_j + \beta_j x_{kj} + e_{kj}$$

It can be seen from the above formula that the result of $y_{kj}$ is affected by three levels of changes of $\alpha_j$, $\beta_j$, and $e_{kj}$, thus constituting a multilevel accumulation model.

The derivation process of the calculation model for the individual dust exposure amount of respiration dust is as follows.

A linear relational expression of the cumulative dust exposure amount of the operators on the mine operating surface is as shown in the following formula (1):

$$d = Q \times C \times t \tag{1}$$

in the formula (1), d is the dust exposure amount of respiration dust in mg;

Q is the individual respiration flow in L/min;
C is a separate respiration dust concentration value in mg/m$^3$; and
t is the time of the operator at the measurement point in s.

The formula (1) can be used for fixed-point sampling of respiration dust by an individual sampler, the flow of Q is constant, and the time of t is a work shift. However, the formula cannot be used for calculating the individual dust exposure amount of the operator, because the influence of humidity, wind speed and the like on-site need to be considered.

Then, Q, C and t are analyzed according to formula (1). Combining with the multilevel mathematical model, the multilevel mathematical models of Q, C and t of the k-th operator at the j point position on the operating surface are respectively obtained by assuming the factors such as humidity, wind speed, onsite concentration and the personnel space-time trajectory as implicit multilevel variables.

$$Q_{kj} = \alpha_j + \beta_j x_{kj} + e_{kj} \tag{2}$$

$$C_{kj} = \alpha_j + \beta_j x_{kj} + e_{kj} \tag{3}$$

$$t_{kj} = \alpha_j + \beta_j x_{kj} + e_{kj} \tag{4}$$

Based on the formula (1), the mathematical calculation model for the individual dust exposure amount of respiration dust of the k-th operator on the operating surface can be derived, as shown in formula (5).

$$D = \sum_{j=0}^{n} Q_{kj} C_{kj} t_{kj} \tag{5}$$

In a preferred embodiment of the present invention, the method for obtaining the wind flow distribution law of the operating surface on-site is as follows:

> obtaining the wind flow distribution law of the operating surface based on wind speed data and position information provided by wind speed sensors disposed on the operating surface, and wherein
> the number of the wind speed sensors is plural, which are evenly or unevenly disposed on a windward side and a leeward side of the operating surface, at a wind flow interference site and within an operating range of the operator.

The wind flow distribution law provides an on-site and real-time wind speed data basis for the acquisition of the distribution law of respiration dust concentration of the operating surface.

In the present embodiment, since the wind flow is blown from a wind inlet side to a wind return side, wind flow measurement points should be arranged in the areas of the windward side and the leeward side, so as to accurately grasp the change of flow state of the wind flow. Since the wind flow is subjected to a hydraulic bracket and the like during the flow process, the wind flow measurement points are arranged at the wind flow interference sites on the upper part and the lower part in a section of the operating surface. In order to ensure that the measured data of wind flow rate and flow velocity of the operating surface on-site are reliable and scientific, a plurality of points should be selected within the operating range of the operator for measurement. Finally an arithmetic or geometric mean value is figured out to obtain an average wind speed at the workplace. The wind speed sensor can be a wind speed detector selectively, which may be a two-way wind speed detector developed by the Chongqing Research Institute Co., Ltd of China Coal Technology & Engineering Group, the wind speed measurement range of which is (0.4-15) m/s, and the resolution of which is 0.1 m/ s. The air volume measurement range of the detector is as follows: the air volume value of the measurement point is displayed in real-time according to the cross sectional area of a roadway, with the maximum range of 750 m$^3$/s. The wind speed detector can upload time information, so that the change characteristics of the wind flow on the operating surface can be acquired at any time. The position of the wind speed detector can be numbered in advance, thus the data uploaded by the wind speed detector would include the position number of the wind speed detector, and the position information of the geographic location of the wind speed detector can be obtained according to the position number thereof.

In a preferred embodiment of the present invention, the number of the respiration dust concentration sensors is plural, which are evenly or unevenly disposed in an area with relatively even dust diffusion on the operating surface, at fixed operating places and within the operating range.

In the present embodiment, the following three principles must be followed when the measurement points of respiration dust concentration on the operating surface is selected.

(1) The measurement points should be in the area with relatively even dust diffusion such as the leeward side or the wind return side.

(2) The operating places operated by onsite workers are relatively fixed, and the measurement points should be located at places where the workers frequently operate or stay.

(3) A number of evenly or unevenly distributed points are selected within the operating range of the workers for measurement.

In the present embodiment, the measurement points of the respiration dust concentration are reasonably arranged, so that the distribution law of the respiration dust concentration of the operating surface can reflect the actual situation as accurately and completely as possible. The respiration dust concentration sensor can be selected from the respiration dust concentration sensor developed by Chongqing Research Institute Co., Ltd of China Coal Technology & Engineering Group, which can upload the respiration dust concentration and the measuring time.

In a preferred embodiment of the present invention, the respiration dust concentration sensor is periodically calibrated, and the calibration process includes:

a standardizing step: standardizing an individual respiration dust detection device by using an individual respiration dust sampler,
a connecting step: carrying, by the operator, the standardized individual respiration dust detection device to the position where the respiration dust concentration sensor is located, and establishing data communication with the respiration dust concentration sensor; and
a calibration step: comparing the onsite detected value of the individual respiration dust detection device with that of the respiration dust concentration sensor, and calibrating the respiration dust concentration sensor based on the detected value of the individual respiration dust detection device.

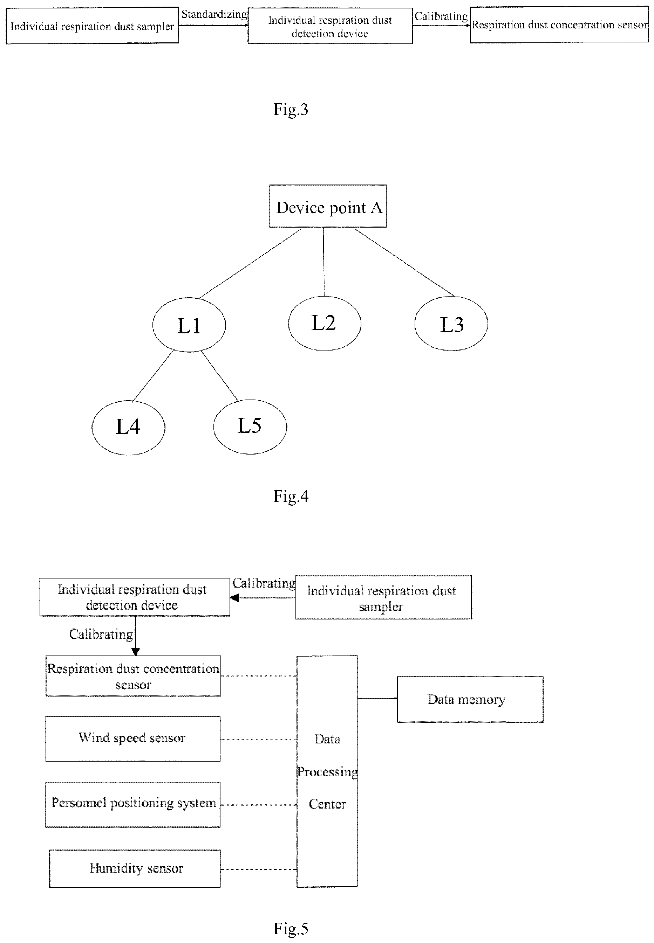

In the present embodiment, Fig.3 is a flow diagram of an error correction process of the respiration dust concentration sensor. The respiration dust concentration sensor is installed on the operating surface with a complicated environment, and thus is subjected to the factors such as onsite high humidity, which causes a measured value error of the respiration dust concentration easy to deviate from a normal range, thus affecting the accuracy of the calculation model for the individual dust exposure amount of respiration dust. In the present embodiment, the individual respiration dust sampler adopts the CCX2.0 type individual respiration dust sampler developed by the Chongqing Research Institute Co., Ltd of China Coal Technology & Engineering Group, the flow accuracy of which is 1%. The individual respiration dust detection device should be of high stability and have small error that can adopt the PDM3700 type individual dust detector of the American Thermoelectric Company, the measurement range of which is (0-200) mg/m$^3$, the error of which is $\pm15\%$, and the flow error of which is $\pm2\%$. The operator wears the individual respiration dust detection device to the location where the respiration dust concentration sensor is installed, and the two devices can communicate with each other in a wireless manner.

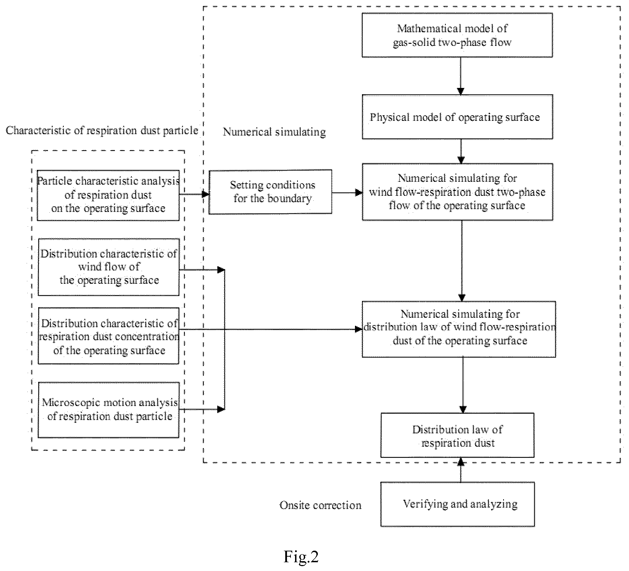

In a preferred embodiment of the present invention, Fig. 2 shows a detailed flow diagram of obtaining the distribution law of respiration dust concentration in a specific embodiment of the present invention. The obtaining process of the distribution law of respiration dust concentration of the operating surface includes the following steps.

S1: establishing a two-phase flow mathematical model of the wind flow and the respiration dust and a physical model of the operating surface.

The wind flow is regarded as a continuous phase of gas phase, the respiration dust is regarded as a dispersed phase of solid phase, and the two phases penetrate and interact with each other. The gas phase can be calculated by using the Eulerina method. Particle tracking can be performed on the solid phase by using the Lagrangian method. A two-phase turbulence flow model of the wind flow and the respiration dust is established.

Gas phase momentum equations:

$$\frac{\partial}{\partial t}(\rho_g v_{gx}) + \frac{\partial}{\partial x}(\rho_g v_{gx} v_{gx}) + \frac{\partial}{\partial y}(\rho_g v_{gy} v_{gx}) + \frac{\partial}{\partial z}(\rho_g v_{gz} v_{gx}) = -\frac{\partial p_g}{\partial x} + \frac{\partial}{\partial x}\tau_{gxx} + \frac{\partial}{\partial y}\tau_{gyx} + \frac{\partial}{\partial z}\tau_{gzx} + \rho_g F_{gx} + \sum_K F_{gpKx} + v_{gx}S$$

$$\frac{\partial}{\partial t}(\rho_g v_{gy}) + \frac{\partial}{\partial x}(\rho_g v_{gx} v_{gy}) + \frac{\partial}{\partial y}(\rho_g v_{gy} v_{gy}) + \frac{\partial}{\partial z}(\rho_g v_{gz} v_{gy}) = -\frac{\partial p_g}{\partial y} + \frac{\partial}{\partial x}\tau_{gxy} + \frac{\partial}{\partial y}\tau_{gyy} + \frac{\partial}{\partial z}\tau_{gzy} + \rho_g F_{gy} + \sum_K F_{gpKy} + v_{gy} S$$

$$\frac{\partial}{\partial t}(\rho_g v_{gz}) + \frac{\partial}{\partial x}(\rho_g v_{gx} v_{gz}) + \frac{\partial}{\partial y}(\rho_g v_{gy} v_{gz}) + \frac{\partial}{\partial z}(\rho_g v_{gz} v_{gz}) = -\frac{\partial p_g}{\partial z} + \frac{\partial}{\partial x}\tau_{gxz} + \frac{\partial}{\partial y}\tau_{gyz} + \frac{\partial}{\partial z}\tau_{gzz} + \rho_g F_{gz} + \sum_K F_{gpKz} + v_{gz} S$$

Particle phase momentum equations:

$$\frac{\partial}{\partial t}(\rho_{pK} v_{pKx}) + \frac{\partial}{\partial x}(\rho_{pK} v_{gx} v_{pKx}) + \frac{\partial}{\partial y}(\rho_{pK} v_{gy} v_{pKx}) + \frac{\partial}{\partial z}(\rho_{pK} v_{gz} v_{pKx}) = -\frac{\partial p_{pK}}{\partial x} + \frac{\partial}{\partial x}\tau_{pKxx} + \frac{\partial}{\partial y}\tau_{pKyx} + \frac{\partial}{\partial z}\tau_{pKzx} + \rho_{pK} F_{pKx} + J_{pKx} + v_{gx} S_{pK}$$

$$\frac{\partial}{\partial t}(\rho_{pK} v_{pKy}) + \frac{\partial}{\partial x}(\rho_{pK} v_{gx} v_{pKy}) + \frac{\partial}{\partial y}(\rho_{pK} v_{gy} v_{pKy}) + \frac{\partial}{\partial z}(\rho_{pK} v_{gz} v_{pKy}) = -\frac{\partial p_{pK}}{\partial y} + \frac{\partial}{\partial x}\tau_{pKxy} + \frac{\partial}{\partial y}\tau_{pKyy} + \frac{\partial}{\partial z}\tau_{pKzy} + \rho_{pK} F_{pKy} + J_{pKy} + v_{gy} S_{pK}$$

$$\frac{\partial}{\partial t}(\rho_{pK} v_{pKz}) + \frac{\partial}{\partial x}(\rho_{pK} v_{gx} v_{pKz}) + \frac{\partial}{\partial y}(\rho_{pK} v_{gy} v_{pKz}) + \frac{\partial}{\partial z}(\rho_{pK} v_{gz} v_{pKz}) = -\frac{\partial p_{pK}}{\partial z} + \frac{\partial}{\partial x}\tau_{pKxz} + \frac{\partial}{\partial y}\tau_{pKyz} + \frac{\partial}{\partial z}\tau_{pKzz} + \rho_{pK} F_{pKz} + J_{pKz} + v_{gz} S_{pK}$$

Gas phase energy equations:

$$\frac{\partial}{\partial t}(\rho_g c_{pg} T_g) + \frac{\partial}{\partial x}(\rho_g v_{gx} c_{pg} T_g) - \frac{\partial p_g}{\partial t} = -\frac{\partial}{\partial x}(\lambda_g \frac{\partial T_g}{\partial x}) + S c_{pg} T_g - \sum_K n_{pK} Q_K - Q_{rg}$$

$$\frac{\partial}{\partial t}(\rho_g c_{pg} T_g) + \frac{\partial}{\partial y}(\rho_g v_{gy} c_{pg} T_g) - \frac{\partial p_g}{\partial t} = -\frac{\partial}{\partial y}(\lambda_g \frac{\partial T_g}{\partial y}) + S c_{pg} T_g - \sum_K n_{pK} Q_K - Q_{rg}$$

$$\frac{\partial}{\partial t}(\rho_g c_{pg} T_g) + \frac{\partial}{\partial z}(\rho_g v_{gz} c_{pg} T_g) - \frac{\partial p_g}{\partial t} = -\frac{\partial}{\partial z}(\lambda_g \frac{\partial T_g}{\partial z}) + S c_{pg} T_g - \sum_K n_{pK} Q_K - Q_{rg}$$

Particle phase energy equations:

$$\frac{\partial}{\partial t}(\rho_{pK} c_{pK} T_{pK}) + \frac{\partial}{\partial x}(\rho_{pK} v_x c_{pK} T_{pK}) = \frac{\partial}{\partial x}(\lambda_{pK} \frac{\partial T_{pK}}{\partial x}) + S_{pK} c_{pg} T_g + n_{pK}(Q_K - Q_{rK})$$

$$\frac{\partial}{\partial t}(\rho_{pK} c_{pK} T_{pK}) + \frac{\partial}{\partial y}(\rho_{pK} v_y c_{pK} T_{pK}) = \frac{\partial}{\partial y}(\lambda_{pK} \frac{\partial T_{pK}}{\partial y}) + S_{pK} c_{pg} T_g + n_{pK}(Q_K - Q_{rK})$$

$$\frac{\partial}{\partial t}(\rho_{pK} c_{pK} T_{pK}) + \frac{\partial}{\partial z}(\rho_{pK} v_z c_{pK} T_{pK}) = \frac{\partial}{\partial z}(\lambda_{pK} \frac{\partial T_{pK}}{\partial z}) + S_{pK} c_{pg} T_g + n_{pK}(Q_K - Q_{rK})$$

Wherein $\rho$ is the fluid density in kg/m$^3$, $v$ is the fluid velocity in m/s, $p$ is the fluid pressure in Pa, $t$ is the time in s, S is the source term in kg/m$^3$, $\tau$ is the shear force in N/m$^3$, F is the volume force in N/m$^3$, $c_p$ is the constant pressure specific heat in W*s/kg*K, $\lambda$ is the heat conductivity coefficient in W/m*K, n is the number of particles, $Q_K$ is the heat taken away by the K-th phase particles from the fluid due to heat convection and its unit is W*s/m, $Q_r$ is the radiant heat in W*s/m, $J_{pK}$ is the component force of the diffusion flow of the K-th phase particles and its unit is N/m$^3$, $F_{gpK}$ is the resultant force of a particle phase acting on a fluid phase and its unit is N/m$^3$. The subscripts in the parameters mean that: g represents the gas phase, p represents the particle phase and K represents the K-th phase particles.

In the present embodiment, in order to improve the aesthetics of the physical model and to facilitate the numerical simulation processing, it is necessary to simplify the machine devices and components in the operating surface, such as a coal cutter and the like is simplified as a large cuboid, an engine arm and a roller are simplified as cylinders, a cable is simplified as a slender cuboid, and the hydraulic bracket is simplified as a cuboid with an inclination angle.

S2: performing numerical simulation to obtain a numerical simulation result of the distribution law of the respiration dust concentration based on the two-phase flow mathematical model of the wind flow and the respiration dust and the physical model of the operating surface.

In the present embodiment, before numerical simulating, it is necessary to define conditions for the numerical simulation parameters that are set reasonably. For example, an inlet condition of the model can adopt a velocity inlet, with the flow velocity and direction of each phase being input. For example, a wall surface in the model is defined as impervious and non-invasive, and the velocity of each phase is zero. For example, the directions are set to be equal at the symmetry axis in the model. And for example, an exit boundary condition in the model is a free surface, and the parameters of each phase are fully developed.

In the present embodiment, a method for numerical solution can adopt the method of two-dimensional axisymmetric steady state solution, the pressure-velocity coupling is processed by a SIMPLES method, and a discrete format adopts a finite volume method.

S3: obtaining a distribution characteristic of the respiration dust concentration of the operating surface based on data of respiration dust concentration provided by the respiration dust concentration sensor disposed on the operating surface, including the following steps:

obtaining wind speed data based on the wind flow distribution law of the operating surface,
verifying and correcting the numerical simulation result of the distribution law of the respiration dust concentration by using the distribution characteristic of the respiration dust concentration of the operating surface and the wind speed data, and obtaining the distribution law of the respiration dust concentration of the operating surface.

In the present embodiment, the specific form of the distribution characteristic of the respiration dust concentration of the operating surface may be an array form. The first element of the array is the position information, that is, the position information of the respiration dust concentration sensor, which can be obtained by the feedback of the respiration dust concentration sensor with a positioning component. The second element of the array is the data of the respiration dust concentration. Based on the distribution characteristic of the respiration dust concentration of the operating surface and the wind speed data provided by the wind flow distribution law, a correction coefficient is obtained by using the maximum principle of correlation coefficient between the numerical simulation result of the distribution law of the respiration dust concentration and detected values, to correct the numerical simulation result of the distribution law of the respiration dust concentration.

In a preferred embodiment of the present invention, the personnel space-time trajectory is obtained according to the positions of the operators reported by a personnel positioning system and the stay time at the positions.

The personnel positioning system includes a plurality of positioning receivers carried by the operators and a device point for receiving information sent by the positioning receivers. A topological relationship between the device point and the positioning receivers is a tree structure. And, topological relationships among the positioning receivers include points and arc segments, arc segments and arc segments, and arc segments and points.

Fig.4 shows a schematic diagram of a tree structure of the personnel positioning system in a specific embodiment of the present invention. The personnel positioning system includes a plurality of positioning receivers carried by the operators and a device point for receiving information sent by the positioning receivers. A topological relationship between the device point and the positioning receivers is a tree structure. And, topological relationships among the positioning receivers include points and arc segments, arc segments and arc segments, and arc segments and points.

In the present embodiment, the positioning receivers include a positioning receiver L1, a positioning receiver L2, a positioning receiver L3, a positioning receiver L4 and a positioning receiver L5, and a device point A for receiving information sent by the positioning receivers. The topological relationship between the device point A and the positioning receivers is the tree structure. The movement trajectory of the operator is calculated through the topological relationship, and errors brought by the personnel positioning system can be well controlled through the movement trajectory of the operator in combination with the data value measured by the positioning system. The topological relationships among the positioning receivers include points and arc segments, arc segments and arc segments, and arc segments and points. Based on the tree structure, hierarchical traversal is performed, and a confidence interval of the positioning information is obtained according to the real-time state information of the two receivers, so that the personnel positioning data are more accurate.

In the present embodiment, the specific form of the personnel space-time trajectory can be in the form of a plurality of arrays stored in a data server. Each array corresponds to one operator. The first element in the array represents a position of the operator, and the second element represents the stay time of the operator at the position.

In a preferred embodiment of the present invention, the method further includes a step of performing onsite correction on the calculation model for the individual dust exposure amount of respiration dust, and the step includes: using a single or multiple individual respiration dust detection devices to continuously detect at the position where the respiration dust concentration sensor is arranged on the operating surface for a time $t_1$, and correcting the calculation model for the individual dust exposure amount of respiration dust by using the detected value.

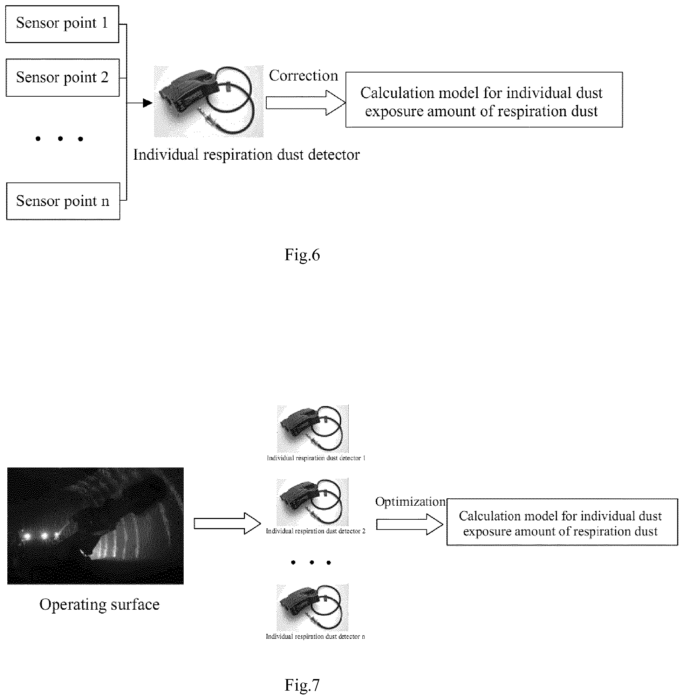

In the present embodiment, Fig. 6 shows a schematic diagram of multi-point corrections for a single device. The individual respiration dust detection device can adopt the PDM3700 type individual dust detector of the American Thermoelectric Company, the measurement range of which is (0-200) mg/m$^3$, the error of which is $\pm 15\%$, and the flow error of which is $\pm 2\%$. The time t1 can be one hour or longer. The dust exposure amount is sequentially measured at positions where the wind speed sensor and/or the respiration dust concentration sensor are disposed on the operating surface. All the measured values are compared with the values calculated by the calculation model for the individual dust exposure amount of respiration dust at the positions. The correction coefficient is obtained according to the maximum principle of correlation coefficient between the measured values and the calculated values, to correct the calculation model for the individual dust exposure amount of respiration dust. The calculation deviation caused by the errors of the distribution law of the respiration dust concentration to the calculation model is eliminated.

In a preferred embodiment of the present invention, the method further includes a step of optimizing the calculation model for the individual dust exposure amount of respiration dust, and the step includes:
respectively carrying, by a plurality of operators, the individual respiration dust detection devices to operate for at least one work shift on the operating surface, and optimizing the calculation model for the individual dust exposure amount of respiration dust by using the measured values of the plurality of individual respiration dust detection devices.

In the present embodiment, Fig.7 shows a schematic diagram of an optimization. The measured values of the plurality of individual respiration dust detection devices are compared with the calculated values of the calculated model. An optimization coefficient is obtained according to the maximum principle of correlation coefficient between the measured values and the calculated values, to optimize the calculated value of the calculation model for the individual dust exposure amount of respiration dust. The calculation deviation caused by the error of the personnel space-time trajectory to the calculation model is eliminated.

In a preferred embodiment of the present invention, the establishment foundation of the calculation model for the individual dust exposure amount of respiration dust further includes an individual separation efficiency law. The calculation model for the individual dust exposure amount of respiration dust is as follows:

$$D = \sum_{j=0}^{n} \eta_{kj} Q_{kj} C_{kj} t_{kj}$$

Wherein $\eta_{kj}$ is an individual separation efficiency coefficient of the k-th operator at the j-th point position on the operating surface, and is obtained based on the individual separation efficiency law.

The process of obtaining the individual separation efficiency law includes the following steps:

an establishment step of a physical model: establishing a separation physical model of a human body respiration dust based on the gas-solid two-phase flow theory,
a numerical simulation step performed based on the separation physical model of the human body respiration dust: numerical simulation of different environmental parameters and the separation efficiency of the separation physical model of the human body respiration dust in a polydisperse state being performed, and obtaining a numerical simulation result of the individual separation efficiency law;
an onsite measuring step: performing onsite sampling and laboratory analysis to process on particle size separation characteristics before and after polydisperse state separation of the dust under the different environmental parameters, and obtaining onsite sampling separation efficiency characteristics; and
a comparison processing step: performing comparative analysis between the numerical simulation result of the individual separation efficiency law and the onsite sampling separation efficiency characteristics, and correcting the numerical simulation result of the individual separation efficiency law according to the analysis result to obtain the individual separation efficiency law.

The physical model is created for a human body separator to facilitate a numerical simulation. After the comparative analysis is performed between the numerical simulation result of the physical model and the onsite sampling result, the numerical simulation result is corrected to obtain an individual separation difference law of the operator based on onsite actual conditions, which compensates and corrects the errors of the individual separation efficiency so that the condition of the actual individual separation efficiency of the operator can be completely reflected.

In the present embodiment, the individual operator is actually a natural respiration dust separator conforming to the BMRC curve, the separation efficiency of which is affected by environmental factors such as onsite humidity, wind speed and polydisperse state of the respiration dust, as well as affected by individual factors such as individual respiration resistance and the like. The high humidity would cause the respiration dust in the polydisperse state to coagulate and bond or the like, so that the particle size of the respiration dust would change, which directly affects the respiration separation efficiency of the individual operator. The wind speed and the individual respiration resistance are inconsistent, resulting in inconsistent flow of the individual respiration separation, which also directly affects the respiration separation efficiency of the individual operator. In the establishment step of the physical model, the separation physical model of the human body respiration dust can be in the form of a dust separator.

The high humidity would cause the respiration dust in the polydisperse state to coagulate and bond or the like, so that the particle size of the respiration dust will change, which directly affects the respiration separation efficiency of the individual worker. The wind speed affects the individual respiration resistance, resulting in that the flow of the individual respiration separation is inconsistent, and which also directly affects the respiration separation efficiency of the individual operator. Thus, the dust exposure amount of respiration dust of the individual operator is not like the direct absorption and reception of the respiration dust in a single disperse state in a laboratory, and is affected by many complicated factors as aforementioned. Therefore, in the step of the numerical simulation based on the physical model, it is necessary to perform the numerical simulation under different environmental parameters and different individual respiration resistances, especially under the environment with high humidity and high wind speed, to obtain a law of the individual separation efficiency under different environmental factors. The law is the numerical simulation result, which has an error with the actual condition. Therefore, the onsite detecting step of sampling and analyzing on-site the respiration dust in the polydisperse state under the same environment and impact factors, is performed to obtain an onsite particle size separation characteristics. A separation efficiency correction coefficient can be obtained according to the maximum principle of correlation coefficient between the onsite measured value and the numerical simulation result, to compensate and correct the error of the numerical simulation result of the individual separation efficiency law, so that the obtained individual separation efficiency law can completely reflect the real individual separation efficiency condition of the operator.

The present invention provides a system for measuring a cumulative dust exposure amount of respiration dust of a mine operator. Fig. 5 shows a block diagram of the system for measuring the cumulative dust exposure amount of respiration dust of the mine operator in a specific embodiment of the present invention. The measuring system includes a data processing center, respiration dust concentration sensors, wind speed sensors, humidity sensors, a personnel positioning system, a data memory and a calibration instrument, wherein the respiration dust concentration sensors, the wind speed sensors, the humidity sensors and the personnel positioning system are in wireless connection with data processing center, and the data memory is in wired connection with the data processing center.

The storage content of the data memory includes a personnel space-time trajectory of all operators and a distribution law of respiration dust concentration of an operating surface.

The number of the respiration dust concentration sensors is plural, which are evenly or unevenly disposed in an area with relatively even dust diffusion of the operating surface, at fixed operating places and within the operating range.

The number of wind speed sensors is plural, which are evenly or unevenly disposed on a windward side and a leeward side of the operating surface, at a wind flow interference site and within the operating range of the operators.

The number of humidity sensors is plural, which are evenly or unevenly disposed on each of the operating surfaces.

The personnel positioning system includes a plurality of positioning receivers carried by the operators and a device point for receiving information sent by the positioning receivers. A topological relationship between the device point and the positioning receivers is a tree structure. Topological relationships among the positioning receivers include points and arc segments, arc segments and arc segments, and arc segments and points.

The calibration instrument includes an individual respiration dust sampler and an individual respiration dust detection device for calibrating the respiration dust concentration sensors.

The data processing center obtains the distribution law of the respiration dust concentration and the personnel space-time trajectory respectively based on the data output by the respiration dust concentration sensors, the wind speed sensors, the personnel positioning system and the humidity sensors, and combines a calculation model for the individual dust exposure amount of respiration dust to calculate the cumulative dust exposure amount of respiration dust of the operators.

In the present embodiment, the data processing center includes a computer or an application server having fast computing capability, and a wireless receiving module for wirelessly receiving the information of the sensors. The data memory may further store the cumulative dust exposure amounts of respiration dust of all operators in a mine, and may also be a data server for providing data for an external third party. The personnel positioning system carried by the operators sends the positions and the current time in real time. After receiving the data, the data processing center generates the personnel space-time trajectory and stores the personnel space-time trajectory in the data memory in the form of an array according to the serial numbers of the operators, which facilitates extracting the data momentarily during the calculation process.

In the description of the present specification, the description with reference to the terms of "one embodiment", "some embodiments", "example", "specific example" or "some examples" and the like means that specific features, structures, materials or characteristics described in combination with the embodiments or examples are included in at least one embodiment or example of the present invention. In the present specification, the schematic representation of the above terms does not necessarily mean the same embodiment or example. Furthermore, the described specific features, structures, materials or characteristics can be combined in a suitable manner in any one or more embodiments or examples.

Although the embodiments of the present invention have been shown and described, scope of the present invention is defined by the claims .

**Claims**

1. A computer-implemented method for calculating a cumulative dust exposure amount of respiration dust of a mine operator, comprising the following steps:

    obtaining a distribution law of respiration dust concentration of an operating surface based on a wind flow distribution law of the operating surface on-site and data output by a respiration dust concentration sensor arranged on the operating surface,
    creating a calculation model for an individual dust exposure amount of respiration dust based on a multilevel accumulative mathematical model in combination with the distribution law of respiration dust concentration of the operating surface and a personnel space-time trajectory of mine operations, and
    calculating the cumulative dust exposure amount of respiration dust of the mine operator by using the calculation model for the individual dust exposure amount of respiration dust,
    wherein the calculation model for the individual dust exposure amount of respiration dust is as follows:

$$D = \sum_{j=0}^{n} Q_{kj} C_{kj} t_{kj}$$

    wherein D is the individual dust exposure amount of respiration dust,
    k is the serial number of the operator on the operating surface, and $k \geq 1$,
    n is the number of position points of the operator in the personnel space-time trajectory on the operating surface,
    j is the sequence number of the position points in the personnel space-time trajectory, and $1 \leq j \leq n$,
    $t_{kj}$ is a stay time of the k-th operator at the j-th point position and is obtained based on the individual space-time trajectory,
    $Q_{kj}$ is an individual respiratory flow of the k-th operator at the j-th point position, and
    $C_{kj}$ is an average respiration dust concentration value of the k-th operator at the j-th point position within a time $\Delta t$ and is obtained based on the distribution law of respiration dust concentration,
    wherein the process of obtaining the distribution law of respiration dust concentration of the operating surface comprises the following steps:

    S1: establishing a two-phase flow mathematical model of the wind flow and the respiration dust and a physical model of the operating surface; wherein the two-phase flow mathematical model of the wind flow and the respiration dust comprises:

    gas phase momentum equations:

$$\frac{\partial}{\partial t}(\rho_g v_{gx}) + \frac{\partial}{\partial x}(\rho_g v_{gx} v_{gx}) + \frac{\partial}{\partial y}(\rho_g v_{gy} v_{gx}) + \frac{\partial}{\partial z}(\rho_g v_{gz} v_{gx}) = -\frac{\partial p_g}{\partial x} + \frac{\partial}{\partial x}\tau_{gx} + \frac{\partial}{\partial y}\tau_{gx} + \frac{\partial}{\partial z}\tau_{gx} + \rho_g F_{gx} + \sum_{K} F_{gpKx} + v_{gx}S$$

$$\frac{\partial}{\partial t}(\rho_g v_{gy}) + \frac{\partial}{\partial x}(\rho_g v_{gx} v_{gy}) + \frac{\partial}{\partial y}(\rho_g v_{gy} v_{gy}) + \frac{\partial}{\partial z}(\rho_g v_{gz} v_{gy}) = -\frac{\partial p_g}{\partial y} + \frac{\partial}{\partial x}\tau_{gxy} + \frac{\partial}{\partial y}\tau_{gyy} + \frac{\partial}{\partial z}\tau_{gzy} + \rho_g F_{gy} + \sum_{K} F_{gpKy} + v_{gy}S$$

$$\frac{\partial}{\partial t}(\rho_g v_{gz}) + \frac{\partial}{\partial x}(\rho_g v_{gx} v_{gz}) + \frac{\partial}{\partial y}(\rho_g v_{gy} v_{gz}) + \frac{\partial}{\partial z}(\rho_g v_{gz} v_{gz}) = -\frac{\partial p_g}{\partial z} + \frac{\partial}{\partial x}\tau_{gxz} + \frac{\partial}{\partial y}\tau_{gyz} + \frac{\partial}{\partial z}\tau_{gzz} + \rho_g F_{gz} + \sum_K F_{gpKz} + v_{gz}S$$

particle phase momentum equations:

$$\frac{\partial}{\partial t}(\rho_{pK} v_{pKx}) + \frac{\partial}{\partial x}(\rho_{pK} v_{gx} v_{pKx}) + \frac{\partial}{\partial y}(\rho_{pK} v_{gy} v_{pKx}) + \frac{\partial}{\partial z}(\rho_{pK} v_{gz} v_{pKx}) = -\frac{\partial p_{pK}}{\partial x} + \frac{\partial}{\partial x}\tau_{pKxx} + \frac{\partial}{\partial y}\tau_{pKyx} + \frac{\partial}{\partial z}\tau_{pKzx} + \rho_{pK} F_{pKx} + J_{pKx} + v_{gx}S_{pK}$$

$$\frac{\partial}{\partial t}(\rho_{pK} v_{pKy}) + \frac{\partial}{\partial x}(\rho_{pK} v_{gx} v_{pKy}) + \frac{\partial}{\partial y}(\rho_{pK} v_{gy} v_{pKy}) + \frac{\partial}{\partial z}(\rho_{pK} v_{gz} v_{pKy}) = -\frac{\partial p_{pK}}{\partial y} + \frac{\partial}{\partial x}\tau_{pKxy} + \frac{\partial}{\partial y}\tau_{pKyy} + \frac{\partial}{\partial z}\tau_{pKzy} + \rho_{pK} F_{pKy} + J_{pKy} + v_{gy}S_{pK}$$

$$\frac{\partial}{\partial t}(\rho_{pK} v_{pKz}) + \frac{\partial}{\partial x}(\rho_{pK} v_{gx} v_{pKz}) + \frac{\partial}{\partial y}(\rho_{pK} v_{gy} v_{pKz}) + \frac{\partial}{\partial z}(\rho_{pK} v_{gz} v_{pKz}) = -\frac{\partial p_{pK}}{\partial z} + \frac{\partial}{\partial x}\tau_{pKxz} + \frac{\partial}{\partial y}\tau_{pKyz} + \frac{\partial}{\partial z}\tau_{pKzz} + \rho_{pK} F_{pKz} + J_{pKz} + v_{gz}S_{pK}$$

gas phase energy equations:

$$\frac{\partial}{\partial t}(\rho_g c_{pg} T_g) + \frac{\partial}{\partial x}(\rho_g v_{gx} c_{pg} T_g) - \frac{\partial p_g}{\partial t} = -\frac{\partial}{\partial x}(\lambda_g \frac{\partial T_g}{\partial x}) + S c_{pg} T_g - \sum_K n_{pK} Q_K - Q_{rg}$$

$$\frac{\partial}{\partial t}(\rho_g c_{pg} T_g) + \frac{\partial}{\partial y}(\rho_g v_{gy} c_{pg} T_g) - \frac{\partial p_g}{\partial t} = -\frac{\partial}{\partial y}(\lambda_g \frac{\partial T_g}{\partial y}) + S c_{pg} T_g - \sum_K n_{pK} Q_K - Q_{rg}$$

$$\frac{\partial}{\partial t}(\rho_g c_{pg} T_g) + \frac{\partial}{\partial z}(\rho_g v_{gz} c_{pg} T_g) - \frac{\partial p_g}{\partial t} = -\frac{\partial}{\partial z}(\lambda_g \frac{\partial T_g}{\partial z}) + S c_{pg} T_g - \sum_K n_{pK} Q_K - Q_{rg}$$

and
particle phase energy equations:

$$\frac{\partial}{\partial t}(\rho_{pK} c_{pK} T_{pK}) + \frac{\partial}{\partial x}(\rho_{pK} v_x c_{pK} T_{pK}) = \frac{\partial}{\partial x}(\lambda_{pK} \frac{\partial T_{pK}}{\partial x}) + S_{pK} c_{pg} T_g + n_{pK}(Q_K - Q_{rK})$$

$$\frac{\partial}{\partial t}(\rho_{pK} c_{pK} T_{pK}) + \frac{\partial}{\partial y}(\rho_{pK} v_y c_{pK} T_{pK}) = \frac{\partial}{\partial y}(\lambda_{pK} \frac{\partial T_{pK}}{\partial y}) + S_{pK} c_{pg} T_g + n_{pK}(Q_K - Q_{rK})$$

$$\frac{\partial}{\partial t}(\rho_{pK} c_{pK} T_{pK}) + \frac{\partial}{\partial z}(\rho_{pK} v_z c_{pK} T_{pK}) = \frac{\partial}{\partial z}(\lambda_{pK} \frac{\partial T_{pK}}{\partial z}) + S_{pK} c_{pg} T_g + n_{pK}(Q_K - Q_{rK})$$

wherein $\rho$ is the fluid density in kg/m³, $v$ is the fluid velocity in m/s, $p$ is the fluid pressure in Pa, $t$ is the time in s, S is the source term in kg/m³, $\tau$ is the shear force in N/m³, F is the volume force in N/m³, $c_p$ is the constant pressure specific heat in W*s/kg*K, $\lambda$ is the heat conductivity coefficient in W/m*K, n is the number of particles, $Q_K$ is the heat taken away by the K-th phase particles from the fluid due to heat convection and its unit is W*s/m, $Q_r$ is the radiant heat in W*s/m, $J_{pK}$ is the component force of the diffusion flow of the K-th phase particles and its unit is N/m³, $F_{gpK}$ is the resultant force of a particle phase acting on a fluid phase and its unit is N/m³, and wherein the subscripts in the parameters mean that: g represents the gas phase, p represents the particle phase and K represents the K-th phase particles;

S2: performing numerical simulation based on the two-phase flow mathematical model of the wind flow and the respiration dust and the physical model of the operating surface, to obtain a numerical simulation result of the distribution law of respiration dust concentration;

S3: obtaining a distribution characteristic of respiration dust concentration of the operating surface based on data of respiration dust concentration provided by the respiration dust concentration sensor disposed on the operating surface;

S4: obtaining wind speed data based on the wind flow distribution law of the operating surface; and

S5: verifying and correcting the numerical simulation result of the distribution law of respiration dust concentration by using the distribution characteristic of respiration dust concentration of the operating surface and the wind speed data, and obtaining the distribution law of respiration dust concentration of the operating surface.

2. The method for calculating the cumulative dust exposure amount of respiration dust of the mine operator according to claim 1, wherein the method for obtaining the wind flow distribution law of the operating surface on-site is as follows:

obtaining the wind flow distribution law of the operating surface based on wind speed data and position information provided by wind speed sensors disposed on the operating surface; wherein the number of the wind speed sensors is plural, which are evenly or unevenly disposed on a windward side and a leeward side of the operating surface, at a wind flow interference site and within an operating range of the operator.

3. The method for calculating the cumulative dust exposure amount of respiration dust of the mine operator according to claim 1, wherein the number of the respiration dust concentration sensors is plural, which are evenly or unevenly disposed in an area with relatively even dust diffusion of the operating surface, at fixed operating places and within an operating range.

4. The method for calculating the cumulative dust exposure amount of respiration dust of the mine operator according to claim 1, wherein the respiration dust concentration sensor is periodically calibrated, and the calibration process comprises:

a standardizing step: standardizing an individual respiration dust detection device by using an individual respiration dust sampler;

a connecting step: carrying, by the operator, the standardized individual respiration dust detection device to the position where the respiration dust concentration sensor is located, and establishing data communication with the respiration dust concentration sensors; and

a calibration step: comparing an onsite detected value of the individual respiration dust detection device with an onsite detected value of the respiration dust concentration sensor, and calibrating the respiration dust concentration sensor based on the detected value of the individual respiration dust detection device.

5. The method for calculating the cumulative dust exposure amount of respiration dust of the mine operator according to claim 1, wherein the personnel space-time trajectory is obtained based on the position of the operator reported by a personnel positioning system and the stay time at the position, and wherein the personnel positioning system comprises a plurality of positioning receivers carried by the operator and a device point for receiving information sent by the positioning receivers, a topological relationship between the device point and the positioning receivers is a tree structure, and topological relationships among the positioning receivers include points and arc segments, arc segments and arc segments, and arc segments and points.

6. The method for calculating the cumulative dust exposure amount of respiration dust of the mine operator according to claim 1, wherein the method further comprises a step of performing an onsite correction on the calculation model for the individual dust exposure amount of respiration dust, and wherein the step comprises:

using a single or multiple individual respiration dust detection devices to continuously detect at the position where the respiration dust concentration sensor is arranged on the operating surface for a time ti, and correcting the calculation model for the individual dust exposure amount of respiration dust by using a detected value.

7. The method for calculating the cumulative dust exposure amount of respiration dust of the mine operator according to claim 1, wherein the method further comprises a step of optimizing the calculation model for the individual dust exposure amount of respiration dust, and wherein the step comprises:

respectively carrying, by a plurality of operators, individual respiration dust detection devices to operate for at least one work shift on the operating surface, and optimizing the calculation model for the individual dust exposure amount of respiration dust by using detected values of the plurality of individual respiration dust detection devices.

8. A system for measuring a cumulative dust exposure amount of respiration dust of a mine operator using the method according to any one of claims 1-7, comprising a data processing center, respiration dust concentration sensors, wind speed sensors, humidity sensors, a personnel positioning system, a data memory and a calibration instrument, wherein the respiration dust concentration sensors, the wind speed sensors, the humidity sensors and the personnel positioning system are in wireless connection with the data processing center, and the data memory is in wired connection with the data processing center; and wherein

the storage content of the data memory comprises a personnel space-time trajectory of all operators on each operating surface of a mine and a distribution law of respiration dust concentration of the operating surface, the number of the respiration dust concentration sensors is plural, which are evenly or unevenly disposed in an area with relatively even dust diffusion of the operating surface, at fixed operating places and within an operating range,

the number of the wind speed sensors is plural, which are evenly or unevenly disposed on a windward side and a leeward side of the operating surface, at a wind flow interference site and within the operating range of the operator,

the number of the humidity sensors is plural, which are evenly or unevenly disposed on the operating surfaces, the personnel positioning system comprises a plurality of positioning receivers carried by the operator and a device point for receiving information sent by the positioning receivers, a topological relationship between the device point and the positioning receivers is a tree structure, and topological relationship among the positioning receivers include points and arc segments, arc segments and arc segments, and arc segments and points, the calibration instrument comprises an individual respiration dust sampler and an individual respiration dust detection device; and

the data processing center obtains the distribution law of respiration dust concentration and the personnel space-time trajectory respectively based on data output by the respiration dust concentration sensors, the wind speed sensors, the personnel positioning system and the humidity sensors, and combines the calculation model for the individual dust exposure amount of respiration dust to calculate the cumulative dust exposure amount of respiration dust of the operator.

**Patentansprüche**

1. Computer-implementiertes Verfahren zum Berechnen einer kumulativen Staubexpositionsmenge von Atmungsstaub eines Bergwerksarbeiters, mit den folgenden Schritten:

Ermitteln eines Verteilungsgesetzes der Atmungsstaubkonzentration einer Betriebsfläche auf der Grundlage eines Windströmungsverteilungsgesetzes der Betriebsfläche vor Ort und von Daten, die von einem auf der Betriebsfläche angeordneten Atmungsstaubkonzentrationssensor ausgegeben werden, Erzeugen eines Berechnungsmodells für eine individuelle Staubexpositionsmenge von Atmungsstaub auf der Grundlage eines mehrstufigen akkumulativen mathematischen Modells in Kombination mit dem Verteilungsgesetz der Atmungsstaubkonzentration der Betriebsfläche und einer Personal-Raum-Zeit-Trajektorie von Bergwerksarbeiten, und Berechnen der kumulativen Staubexpositionsmenge an Atmungsstaub des Bergwerksarbeiters unter Verwendung des Berechnungsmodells für die individuelle Staubexpositionsmenge an Atmungsstaub, wobei das Berechnungsmodell für die individuelle Staubexpositionsmenge von Atmungsstaub wie folgt ist:

$$D = \sum_{j=0}^{n} Q_{kj} C_{kj} t_{kj}$$

wobei D die individuelle Staubexpositionsmenge an Atmungsstaub ist,
k die Seriennummer des Arbeiters auf der Betriebsfläche ist, und k≥1,
n die Anzahl der Positionspunkte des Arbeiters in der Personal-Raum-Zeit-Trajektorie auf der Betriebsfläche ist, j die laufende Nummer der Positionspunkte in der Personal-Raum-Zeit-Trajektorie ist und 1≤j≤n,
$t_{kj}$ die Verweildauer des k-ten Arbeiters an dem j-ten Positionspunkt ist und auf der Grundlage der individuellen Raum-Zeit-Trajektorie ermittelt wird,
$Q_{kj}$ ein individueller Atemfluss des k-ten Arbeiters an der j-ten Positionspunkt ist und

$C_{kj}$ ein durchschnittlicher Atmungsstaubkonzentrationswert des k-ten Arbeiters am j-ten Positionspunkt innerhalb einer Zeit $\Delta t$ ist und auf der Grundlage des Verteilungsgesetzes der Atmungsstaubkonzentration erhalten wird,

wobei das Verfahren zur Gewinnung des Verteilungsgesetzes der Atmungsstaubkonzentration der Betriebsfläche die folgenden Schritte umfasst:

S1: Aufstellen eines mathematischen Zweiphasenströmungsmodells der Windströmung und des Atmungsstaubs und eines physikalischen Modells der Betriebsfläche; wobei das mathematische Zweiphasenströmungsmodell der Windströmung und des Atmungsstaubs umfasst:

Gasphasen-Impuls-Gleichungen:

$$\frac{\partial}{\partial t}(\rho_g v_{gx}) + \frac{\partial}{\partial x}(\rho_g v_{gx} v_{gx}) + \frac{\partial}{\partial y}(\rho_g v_{gy} v_{gx}) + \frac{\partial}{\partial z}(\rho_g v_{gz} v_{gx}) = -\frac{\partial p_g}{\partial x} + \frac{\partial}{\partial x}\tau_{gxx} + \frac{\partial}{\partial y}\tau_{gyx} + \frac{\partial}{\partial z}\tau_{gzx} + \rho_g F_{gx} + \sum_K F_{gpKx} + v_{gx}S$$

$$\frac{\partial}{\partial t}(\rho_g v_{gy}) + \frac{\partial}{\partial x}(\rho_g v_{gx} v_{gy}) + \frac{\partial}{\partial y}(\rho_g v_{gy} v_{gy}) + \frac{\partial}{\partial z}(\rho_g v_{gz} v_{gy}) = -\frac{\partial p_g}{\partial y} + \frac{\partial}{\partial x}\tau_{gxy} + \frac{\partial}{\partial y}\tau_{gyy} + \frac{\partial}{\partial z}\tau_{gzy} + \rho_g F_{gy} + \sum_K F_{gpKy} + v_{gy}S$$

$$\frac{\partial}{\partial t}(\rho_g v_{gz}) + \frac{\partial}{\partial x}(\rho_g v_{gx} v_{gz}) + \frac{\partial}{\partial y}(\rho_g v_{gy} v_{gz}) + \frac{\partial}{\partial z}(\rho_g v_{gz} v_{gz}) = -\frac{\partial p_g}{\partial z} + \frac{\partial}{\partial x}\tau_{gxz} + \frac{\partial}{\partial y}\tau_{gyz} + \frac{\partial}{\partial z}\tau_{gzz} + \rho_g F_{gz} + \sum_K F_{gpKz} + v_{gz}S$$

Teilchenphasen-Impuls-Gleichungen:

$$\frac{\partial}{\partial t}(\rho_{pK} v_{pKx}) + \frac{\partial}{\partial x}(\rho_{pK} v_{gx} v_{pKx}) + \frac{\partial}{\partial y}(\rho_{pK} v_{gy} v_{pKx}) + \frac{\partial}{\partial z}(\rho_{pK} v_{gz} v_{pKx}) = -\frac{\partial p_{pK}}{\partial x} + \frac{\partial}{\partial x}\tau_{pKxx} + \frac{\partial}{\partial y}\tau_{pKyx} + \frac{\partial}{\partial z}\tau_{pKzx} + \rho_{pK} F_{pKx} + J_{pKx} + v_{gx}S_{pK}$$

$$\frac{\partial}{\partial t}(\rho_{pK} v_{pKy}) + \frac{\partial}{\partial x}(\rho_{pK} v_{gx} v_{pKy}) + \frac{\partial}{\partial y}(\rho_{pK} v_{gy} v_{pKy}) + \frac{\partial}{\partial z}(\rho_{pK} v_{gz} v_{pKy}) = -\frac{\partial p_{pK}}{\partial y} + \frac{\partial}{\partial x}\tau_{pKxy} + \frac{\partial}{\partial y}\tau_{pKyy} + \frac{\partial}{\partial z}\tau_{pKzy} + \rho_{pK} F_{pKy} + J_{pKy} + v_{gy}S_{pK}$$

$$\frac{\partial}{\partial t}(\rho_{pK} v_{pKz}) + \frac{\partial}{\partial x}(\rho_{pK} v_{gx} v_{pKz}) + \frac{\partial}{\partial y}(\rho_{pK} v_{gy} v_{pKz}) + \frac{\partial}{\partial z}(\rho_{pK} v_{gz} v_{pKz}) = -\frac{\partial p_{pK}}{\partial z} + \frac{\partial}{\partial x}\tau_{pKxz} + \frac{\partial}{\partial y}\tau_{pKyz} + \frac{\partial}{\partial z}\tau_{pKzz} + \rho_{pK} F_{pKz} + J_{pKz} + v_{gz}S_{pK}$$

Gasphasen-Energi e-Gleichungen:

$$\frac{\partial}{\partial t}(\rho_g c_{pg} T_g) + \frac{\partial}{\partial x}(\rho_g v_{gx} c_{pg} T_g) - \frac{\partial p_g}{\partial t} = -\frac{\partial}{\partial x}(\lambda_g \frac{\partial T_g}{\partial x}) + S c_{pg} T_g - \sum_K n_{pK} Q_K - Q_{rg}$$

$$\frac{\partial}{\partial t}(\rho_g c_{pg} T_g) + \frac{\partial}{\partial y}(\rho_g v_{gy} c_{pg} T_g) - \frac{\partial p_g}{\partial t} = -\frac{\partial}{\partial y}(\lambda_g \frac{\partial T_g}{\partial y}) + S c_{pg} T_g - \sum_K n_{pK} Q_K - Q_{rg}$$

$$\frac{\partial}{\partial t}(\rho_g c_{pg} T_g) + \frac{\partial}{\partial z}(\rho_g v_{gz} c_{pg} T_g) - \frac{\partial p_g}{\partial t} = -\frac{\partial}{\partial z}(\lambda_g \frac{\partial T_g}{\partial z}) + S c_{pg} T_g - \sum_K n_{pK} Q_K - Q_{rg}$$

und
Partikelphasen-Energiegleichungen:

$$\frac{\partial}{\partial t}(\rho_{pK} c_{pK} T_{pK}) + \frac{\partial}{\partial x}(\rho_{pK} v_x c_{pK} T_{pK}) = \frac{\partial}{\partial x}(\lambda_{pK} \frac{\partial T_{pK}}{\partial x}) + S_{pK} c_{pg} T_g + n_{pK}(Q_K - Q_{rK})$$

$$\frac{\partial}{\partial t}(\rho_{pK} c_{pK} T_{pK}) + \frac{\partial}{\partial y}(\rho_{pK} v_y c_{pK} T_{pK}) = \frac{\partial}{\partial y}(\lambda_{pK} \frac{\partial T_{pK}}{\partial y}) + S_{pK} c_{pg} T_g + n_{pK}(Q_K - Q_{rK})$$

$$\frac{\partial}{\partial t}(\rho_{pK} c_{pK} T_{pK}) + \frac{\partial}{\partial z}(\rho_{pK} v_z c_{pK} T_{pK}) = \frac{\partial}{\partial z}(\lambda_{pK} \frac{\partial T_{pK}}{\partial z}) + S_{pK} c_{pg} T_g + n_{pK}(Q_K - Q_{rK})$$

Wobei $\rho$ die Fluiddichte in kg/m$^3$, v die Fluidgeschwindigkeit in m/s, p der Fluiddruck in Pa, t die Zeit in s, S der Quellterm in kg/m$^3$, $\tau$ die Scherkraft in N/m$^3$, F die Volumenkraft in N/m$^3$, $c_p$ die spezifische Wärme bei konstantem Druck in W*s/kg*K, $\lambda$ der Wärmeleitfähigkeitskoeffizient in W/m*K, n die Anzahl der Teilchen ist, $Q_k$ die von den Teilchen der K-ten Phase aufgrund von Wärmekonvektion aus dem Fluid abgeführte Wärme ist und ihre Einheit W*s/m ist, $Q_r$ die Strahlungswärme in W*s/m ist, $J_{pK}$ die Komponentenkraft des Diffusionsstroms der Teilchen der K-ten Phase ist und ihre Einheit N/m$^3$ ist, $F_{gpK}$ die resultierende Kraft einer Teilchenphase, die auf eine Fluidphase wirkt, ist und ihre Einheit N/m$^3$ ist, und wobei die tiefgestellten Buchstaben in den Parametern bedeuten, dass: g für die Gasphase, p für die Partikelphase und K für die K-te Partikelphase steht;

S2: Durchführen einer numerischen Simulation auf der Grundlage des mathematischen Zweiphasenströmungsmodells der Windströmung und des Atmungsstaubs und des physikalischen Modells der Betriebsfläche, um ein numerisches Simulationsergebnis des Verteilungsgesetzes der Atmungsstaubkonzentration zu erhalten;
S3: Erhalten einer Verteilungscharakteristik der Atmungsstaubkonzentration der Betriebsfläche auf der Grundlage von Daten der Atmungsstaubkonzentration, die von dem auf der Betriebsfläche angeordneten Atmungsstaubkonzentrationssensor bereitgestellt werden;
S4: Gewinnen von Windgeschwindigkeitsdaten auf der Grundlage des Windströmungsverteilungsgesetzes der Betriebsfläche; und
S5: Verifizieren und Korrigieren des numerischen Simulationsergebnisses des Verteilungsgesetzes der Atmungsstaubkonzentration unter Verwendung der Verteilungscharakteristik der Atmungsstaubkonzentration der Betriebsfläche und der Windgeschwindigkeitsdaten, und Erhalten des Verteilungsgesetzes der Atmungsstaubkonzentration der Betriebsfläche.

2. Verfahren zum Berechnen der kumulativen Staubexpositionsmenge von Atmungsstaub für den Bergwerksbetreiber nach Anspruch 1, wobei das Verfahren zum Erhalten des Windströmungsverteilungsgesetzes der Betriebsfläche vor Ort wie folgt ist:

Ermitteln des Windströmungs-Verteilungsgesetzes der Betriebsfläche auf der Grundlage von Windgeschwindigkeitsdaten und Positionsinformationen, die von auf der Betriebsfläche angeordneten Windgeschwindigkeitssensoren bereitgestellt werden; wobei
Die Windgeschwindigkeitssensoren an Anzahl mehrere sind, die gleichmäßig oder ungleichmäßig auf einer Luvseite und einer Leeseite der Betriebsfläche, an einem Windströmungsstörungsort und innerhalb eines Arbeitsbereichs des Arbeiters angeordnet sind.

3. Verfahren zum Berechnen der kumulativen Staubexpositionsmenge von Atmungsstaub des Bergwerksarbeiters nach Anspruch 1, wobei die Atmungsstaubkonzentrationssensoren an Anzahl mehrere sind, die gleichmäßig oder ungleichmäßig in einem Bereich mit relativ gleichmäßiger Staubdiffusion der Betriebsfläche, an festen Betriebsorten und innerhalb eines Arbeitsbereichs angeordnet sind.

4. Verfahren zum Berechnen der kumulativen Staubexpositionsmenge von Atmungsstaub des Bergwerksarbeiters gemäß Anspruch 1, wobei der Atmungsstaubkonzentrationssensor periodisch kalibriert wird und der Kalibrierungsprozess umfasst:

einen Kalibrierungsschritt: Kalibrieren einer individuellen Atmungsstaubdetektionsvorrichtung unter Verwendung eines individuellen Atmungsstaubsammlers;

einen Verbindungsschritt: Tragen der standardisierten individuellen Atmungsstaub-Erfassungsvorrichtung durch den Arbeiter zu der Position, an der sich der Atmungsstaub-Konzentrationssensor befindet, und Herstellen einer Datenkommunikation mit den Atmungsstaub-Konzentrationssensoren; und

einen Kalibrierungsschritt: Vergleichen eines vor Ort erfassten Wertes der individuellen Atmungsstaub-Erfassungsvorrichtung mit einem vor Ort erfassten Wert des Atmungsstaub-Konzentrationssensors und Kalibrieren des Atmungsstaub-Konzentrationssensors auf der Grundlage des erfassten Wertes der individuellen Atmungsstaub-Erfassungsvorrichtung.

**5.** Verfahren zum Berechnen der kumulativen Staubexpositionsmenge an Atmungsstaub des Bergwerksarbeiters nach Anspruch 1, wobei die Personal-Raum-Zeit-Trajektorie auf der Grundlage der von einem Personalpositionierungssystem gemeldeten Position des Arbeiters und der Aufenthaltszeit an der Position erhalten wird, und wobei das Personalpositionierungssystem eine Vielzahl von Positionierungsempfängern, die von dem Arbeiter getragen werden, und einen Gerätepunkt zum Empfangen von Informationen, die von den Positionierungsempfängern gesendet werden, umfasst, eine topologische Beziehung zwischen dem Gerätepunkt und den Positionierungsempfängern eine Baumstruktur ist und topologische Beziehungen zwischen den Positionierungsempfängern Punkte und Bogensegmente, Bogensegmente und Bogensegmente und Bogensegmente und Punkte umfassen.

**6.** Verfahren zum Berechnen der kumulativen Staubexpositionsmenge an Atmungsstaub des Bergwerksarbeiters nach Anspruch 1, wobei das Verfahren ferner einen Schritt des Durchführens einer Vor-Ort-Korrektur an dem Berechnungsmodell für die individuelle Staubexpositionsmenge an Atmungsstaub umfasst, und wobei der Schritt umfasst: Verwenden einer einzelnen oder mehrerer individueller Atmungsstaub-Detektionsvorrichtungen, um kontinuierlich an der Position zu detektieren, an der der Atmungsstaub-Konzentrationssensor auf der Arbeitsfläche für eine Zeit $t_1$ angeordnet ist, und Korrigieren des Berechnungsmodells für die individuelle Staubexpositionsmenge von Atmungsstaub unter Verwendung eines detektierten Wertes.

**7.** Verfahren zum Berechnen der kumulativen Staubexpositionsmenge an Atmungsstaub des Bergwerksarbeiters gemäß Anspruch 1, wobei das Verfahren ferner einen Schritt des Optimierens des Berechnungsmodells für die individuelle Staubexpositionsmenge an Atmungsstaub umfasst, und wobei der Schritt umfasst: Tragen von individuellen Atmungsstaub-Detektionsvorrichtungen durch eine Vielzahl von Arbeitern, um für mindestens eine Betriebsschicht auf der Betriebsfläche zu arbeiten, und Optimieren des Berechnungsmodells für die individuelle Staubexpositionsmenge von Atmungsstaub unter Verwendung von detektierten Werten der Vielzahl von individuellen Atmungsstaub-Detektionsvorrichtungen.

**8.** System zum Messen einer kumulativen Staubexpositionsmenge von Atmungsstaub eines Bergwerksarbeiters unter Verwendung des Verfahrens nach einem der Ansprüche 1 bis 7, umfassend ein Datenverarbeitungszentrum, Atmungsstaubkonzentrationssensoren, Windgeschwindigkeitssensoren, Feuchtigkeitssensoren, ein Personalpositionierungssystem, einen Datenspeicher und ein Kalibrierungsinstrument, wobei die Atmungsstaubkonzentrationssensoren, die Windgeschwindigkeitssensoren, die Feuchtigkeitssensoren und das Personalpositionierungssystem in drahtloser Verbindung mit dem Datenverarbeitungszentrum stehen, und der Datenspeicher in drahtgebundener Verbindung mit dem Datenverarbeitungszentrum steht; und wobei

der Speicherinhalt des Datenspeichers eine Personal-Raum-Zeit-Trajektorie aller Arbeiter auf jeder Betriebsfläche eines Bergwerks und ein Verteilungsgesetz der Atmungsstaubkonzentration der Betriebsfläche umfasst, die Anzahl der Atmungsstaubkonzentrationssensoren eine Mehrzahl ist, die gleichmäßig oder ungleichmäßig in einem Bereich mit relativ gleichmäßiger Staubdiffusion der Betriebsfläche, an festen Betriebsorten und innerhalb eines Betriebsbereichs angeordnet sind, die Anzahl der Windgeschwindigkeitssensoren ist eine Mehrzahl, die gleichmäßig oder ungleichmäßig auf einer Luvseite und einer Leeseite der Betriebsfläche, an einem Windströmungsstörungsort und innerhalb des Betriebsbereichs des Arbeiters angeordnet sind, die Anzahl der Feuchtigkeitssensoren, die gleichmäßig oder ungleichmäßig auf den Bedienflächen angeordnet sind, ist mehrfach, das Personenpositionierungssystem eine Vielzahl von Positionierungsempfängern, die von der Bedienungsperson getragen werden, und einen Gerätepunkt zum Empfangen von Informationen, die von den Positionierungsempfängern gesendet werden, umfasst, eine topologische Beziehung zwischen dem Gerätepunkt und den Positionierungsempfängern eine Baumstruktur ist, und die topologische Beziehung zwischen den Positionierungsempfängern Punkte und Bogensegmente, Bogensegmente und Bogensegmente und Bogensegmente und Punkte umfasst, das Kalibrierungsinstrument einen individuellen Atmungsstaubsammler und eine individuelle Atmungsstaub-Erfassungsvorrichtung umfasst; und

das Datenverarbeitungszentrum das Verteilungsgesetz der Atmungsstaubkonzentration und die Raum-Zeit-Trajektorie des Personals jeweils auf der Grundlage von Daten erhält, die von den Atmungsstaubkonzentrationssensoren, den Windgeschwindigkeitssensoren, dem Personalpositionierungssystem und den Feuchtigkeitssensoren ausgegeben werden, und das Berechnungsmodell für die individuelle Staubexpositionsmenge von Atmungsstaub kombiniert, um die kumulative Staubexpositionsmenge von Atmungsstaub des Arbeiters zu berechnen.

## Revendications

1. Procédé mis en œuvre par ordinateur pour calculer une quantité cumulée d'exposition à la poussière de poussière de respiration d'un exploitant minier, comprenant les étapes suivantes :

   obtention d'une loi de distribution de la concentration de poussière de respiration d'une surface de fonctionnement sur la base d'une loi de distribution d'écoulement du vent de la surface de fonctionnement sur site et d'une sortie de données au moyen d'un capteur de concentration de poussière de respiration agencé sur la surface de fonctionnement,
   création d'un modèle de calcul pour une quantité individuelle d'exposition à la poussière de poussière de respiration sur la base d'un modèle mathématique cumulatif à plusieurs niveaux en combinaison avec la loi de distribution de la concentration de poussière de respiration de la surface de fonctionnement et une trajectoire spatio-temporelle du personnel des opérations minières, et
   calcul de la quantité cumulée d'exposition à la poussière de poussière de respiration de l'exploitant minier en utilisant le modèle de calcul pour la quantité individuelle d'exposition à la poussière de poussière de respiration,
   dans lequel le modèle de calcul pour la quantité individuelle d'exposition à la poussière de poussière de respiration se présente comme suit :

$$D = \sum_{j=0}^{n} Q_{kj} C_{kj} t_{kj}$$

   dans laquelle D représente la quantité individuelle d'exposition à la poussière de poussière de respiration,
   k représente le numéro de série de l'exploitant sur la surface de fonctionnement, et $k \geq 1$,
   n représente le nombre de points de position de l'exploitant dans la trajectoire spatio-temporelle du personnel sur la surface de fonctionnement, j représente le numéro de séquence des points de position dans la trajectoire spatio-temporelle du personnel, et $1 \leq j \leq n$,
   $t_{kj}$ représente un temps de séjour du k-ième exploitant à la j-ième position de point et est obtenu sur la base de la trajectoire spatio-temporelle individuelle,
   $Q_{kj}$ représente un écoulement respiratoire individuel du k-ième exploitant à la j-ième position de point, et
   $C_{kj}$ représente une valeur moyenne de concentration de poussière de respiration du k-ième exploitant à la j-ième position de point dans un délai $\Delta t$ et est obtenue sur la base de la loi de distribution de la concentration de poussière de respiration,
   dans lequel le processus d'obtention de la loi de distribution de la concentration de poussière de respiration de la surface de fonctionnement comprend les étapes suivantes :

   S1 : établir un modèle mathématique d'écoulement à deux phases de l'écoulement du vent et la poussière de respiration et un modèle physique de la surface de fonctionnement ; dans lequel le modèle mathématique d'écoulement à deux phases de l'écoulement du vent et la poussière de respiration comprend :

   des équations de quantité de mouvement de phase gazeuse :

$$\frac{\partial}{\partial t}(\rho_g v_{gx}) + \frac{\partial}{\partial x}(\rho_g v_{gx} v_{gx}) + \frac{\partial}{\partial y}(\rho_g v_{gy} v_{gx}) + \frac{\partial}{\partial z}(\rho_g v_{gz} v_{gx}) = -\frac{\partial p_g}{\partial x} + \frac{\partial}{\partial x}\tau_{gx} + \frac{\partial}{\partial y}\tau_{gx} + \frac{\partial}{\partial z}\tau_{gx} + \rho_g F_{gx} + \sum_K F_{gpKx} + v_{gx}S$$

$$\frac{\partial}{\partial t}(\rho_g v_{gy}) + \frac{\partial}{\partial x}(\rho_g v_{gx} v_{gy}) + \frac{\partial}{\partial y}(\rho_g v_{gy} v_{gy}) + \frac{\partial}{\partial z}(\rho_g v_{gz} v_{gy}) = -\frac{\partial p_g}{\partial y} + \frac{\partial}{\partial x}\tau_{gxy} + \frac{\partial}{\partial y}\tau_{gyy} + \frac{\partial}{\partial z}\tau_{gzy} + \rho_g F_{gy} + \sum_K F_{gpKy} + v_{gy}S$$

$$\frac{\partial}{\partial t}(\rho_g v_{gz}) + \frac{\partial}{\partial x}(\rho_g v_{gx} v_{gz}) + \frac{\partial}{\partial y}(\rho_g v_{gy} v_{gz}) + \frac{\partial}{\partial z}(\rho_g v_{gz} v_{gz}) = -\frac{\partial p_g}{\partial z} + \frac{\partial}{\partial x}\tau_{gxz} + \frac{\partial}{\partial y}\tau_{gyz} + \frac{\partial}{\partial z}\tau_{gzz} + \rho_g F_{gz} + \sum_K F_{gpKz} + v_{gz}S$$

des équations de quantité de mouvement de phase particulaire :

$$\frac{\partial}{\partial t}(\rho_{pK} v_{pKx}) + \frac{\partial}{\partial x}(\rho_{pK} v_{gx} v_{pKx}) + \frac{\partial}{\partial y}(\rho_{pK} v_{gy} v_{pKx}) + \frac{\partial}{\partial z}(\rho_{pK} v_{gz} v_{pKx}) = -\frac{\partial p_{pK}}{\partial x} + \frac{\partial}{\partial x}\tau_{pKxx} + \frac{\partial}{\partial y}\tau_{pKyx} + \frac{\partial}{\partial z}\tau_{pKzx} + \rho_{pK} F_{pKx} + J_{pKx} + v_{gx}S_{pK}$$

$$\frac{\partial}{\partial t}(\rho_{pK} v_{pKy}) + \frac{\partial}{\partial x}(\rho_{pK} v_{gx} v_{pKy}) + \frac{\partial}{\partial y}(\rho_{pK} v_{gy} v_{pKy}) + \frac{\partial}{\partial z}(\rho_{pK} v_{gz} v_{pKy}) = -\frac{\partial p_{pK}}{\partial y} + \frac{\partial}{\partial x}\tau_{pKxy} + \frac{\partial}{\partial y}\tau_{pKyy} + \frac{\partial}{\partial z}\tau_{pKzy} + \rho_{pK} F_{pKy} + J_{pKy} + v_{gy}S_{pK}$$

$$\frac{\partial}{\partial t}(\rho_{pK} v_{pKz}) + \frac{\partial}{\partial x}(\rho_{pK} v_{gx} v_{pKz}) + \frac{\partial}{\partial y}(\rho_{pK} v_{gy} v_{pKz}) + \frac{\partial}{\partial z}(\rho_{pK} v_{gz} v_{pKz}) = -\frac{\partial p_{pK}}{\partial z} + \frac{\partial}{\partial x}\tau_{pKxz} + \frac{\partial}{\partial y}\tau_{pKyz} + \frac{\partial}{\partial z}\tau_{pKzz} + \rho_{pK} F_{pKz} + J_{pKz} + v_{gz}S_{pK}$$

des équations d'énergie de phase gazeuse :

$$\frac{\partial}{\partial t}(\rho_g c_{pg} T_g) + \frac{\partial}{\partial x}(\rho_g v_{gx} c_{pg} T_g) - \frac{\partial p_g}{\partial t} = -\frac{\partial}{\partial x}(\lambda_g \frac{\partial T_g}{\partial x}) + S c_{pg} T_g - \sum_K n_{pK} Q_K - Q_{rg}$$

$$\frac{\partial}{\partial t}(\rho_g c_{pg} T_g) + \frac{\partial}{\partial y}(\rho_g v_{gy} c_{pg} T_g) - \frac{\partial p_g}{\partial t} = -\frac{\partial}{\partial y}(\lambda_g \frac{\partial T_g}{\partial y}) + S c_{pg} T_g - \sum_K n_{pK} Q_K - Q_{rg}$$

$$\frac{\partial}{\partial t}(\rho_g c_{pg} T_g) + \frac{\partial}{\partial z}(\rho_g v_{gz} c_{pg} T_g) - \frac{\partial p_g}{\partial t} = -\frac{\partial}{\partial z}(\lambda_g \frac{\partial T_g}{\partial z}) + S c_{pg} T_g - \sum_K n_{pK} Q_K - Q_{rg}$$

et
des équations d'énergie de phase particulaire :

$$\frac{\partial}{\partial t}(\rho_{pK} c_{pK} T_{pK}) + \frac{\partial}{\partial x}(\rho_{pK} v_x c_{pK} T_{pK}) = \frac{\partial}{\partial x}(\lambda_{pK} \frac{\partial T_{pK}}{\partial x}) + S_{pK} c_{pg} T_g + n_{pK}(Q_K - Q_{rK})$$

$$\frac{\partial}{\partial t}(\rho_{pK} c_{pK} T_{pK}) + \frac{\partial}{\partial y}(\rho_{pK} v_y c_{pK} T_{pK}) = \frac{\partial}{\partial y}(\lambda_{pK} \frac{\partial T_{pK}}{\partial y}) + S_{pK} c_{pg} T_g + n_{pK}(Q_K - Q_{rK})$$

$$\frac{\partial}{\partial t}(\rho_{pK} c_{pK} T_{pK}) + \frac{\partial}{\partial z}(\rho_{pK} v_z c_{pK} T_{pK}) = \frac{\partial}{\partial z}(\lambda_{pK} \frac{\partial T_{pK}}{\partial z}) + S_{pK} c_{pg} T_g + n_{pK}(Q_K - Q_{rK})$$

dans lesquelles $\rho$ représente la densité de fluide en kg/m³, $v$ représente la vitesse de fluide en m/s, $p$ représente la pression de fluide en Pa, $t$ représente le temps en s, S représente le terme source en kg/m³, $\tau$ représente la force de cisaillement en N/m³, F représente la force volumique en N/m³, $c_p$ représente la chaleur spécifique à pression constante en W*s/kg*K, $\lambda$ représente le coefficient de conductivité thermique en W/m*K, n représente le nombre de particules, $Q_K$ représente la chaleur emportée par les K-ième particules de phase provenant du fluide en raison de la convection thermique et son unité est le W*s/m, $Q_r$ représente la chaleur rayonnante en W*s/m, $J_{pK}$ représente la force composante du flux de diffusion des K-ième particules de phase et son unité est le N/m$^3$, $F_{gpK}$ représente la force résultante d'une phase particulaire agissant sur une phase de fluide et son unité est le N/m$^3$, et dans lequel les indices dans les paramètres indiquent que : g représente la phase gazeuse, p représente la phase particulaire et K représente les K-ième particules de phase ;

S2 : effectuer une simulation numérique sur la base du modèle mathématique d'écoulement à deux phases de l'écoulement du vent et de la poussière de respiration et du modèle physique de la surface de fonctionnement, afin d'obtenir un résultat de simulation numérique de la loi de distribution de la concentration de poussière de respiration ;

S3 : obtenir une distribution caractéristique de la concentration de poussière de respiration de la surface de fonctionnement sur la base de les données de la concentration de poussière de respiration fournies par le capteur de concentration de poussière de respiration disposé sur la surface de fonctionnement ;

S4 : obtenir des données relatives à la vitesse du vent sur la base de la loi de distribution d'écoulement du vent de la surface de fonctionnement ; et

S5 : vérifier et corriger le résultat de simulation de la loi de distribution de la concentration de poussière de respiration en utilisant la caractéristique de distribution de la concentration de poussière de respiration de la surface de fonctionnement et les données relatives à la vitesse du vent, et obtenir la loi de distribution de la concentration de poussière de respiration de la surface de fonctionnement.

2. Procédé de calcul de la quantité cumulée d'exposition à la poussière de la poussière de respiration de l'exploitant de la mine selon la revendication 1, dans lequel le procédé pour obtenir la loi de distribution d'écoulement du vent de la surface de fonctionnement sur site se présente comme suit :

   obtention de la loi de distribution d'écoulement du vent de la surface de fonctionnement sur la base des données relatives à la vitesse du vent et des informations relatives à la position fournies par des capteurs de vitesse du vent disposés sur la surface de fonctionnement ; dans lequel
   le nombre de capteurs de vitesse du vent est pluriel, qui sont disposés de manière égale ou inégale sur un côté au vent et un côté sous le vent de la surface de fonctionnement, au niveau d'un site d'interférence d'écoulement du vent et dans une plage de fonctionnement de l'exploitant.

3. Procédé de calcul de la quantité cumulée d'exposition à la poussière de la poussière de respiration de l'exploitant de la mine selon la revendication 1, dans lequel le nombre de capteurs de concentration de poussière de respiration est pluriel, qui sont disposés de manière égale ou inégale dans une zone avec une diffusion de poussière relativement uniforme de la surface de fonctionnement, à des lieux d'exploitation fixes et dans une plage de fonctionnement.

4. Procédé de calcul de la quantité cumulée d'exposition à la poussière de la poussière de respiration de l'exploitant de la mine selon la revendication 1, dans lequel le capteur de concentration de poussière de respiration est calibré de manière périodique, et le procédé de calibration comprend :

   une étape de standardisation : standardisation d'un dispositif individuel de détection de poussière de respiration en utilisant un échantillonneur individuel de poussière de respiration ;
   une étape de connexion : le fait d'apporter, par l'exploitant, le dispositif individuel de détection de poussière de respiration standardisé à la position où le capteur de concentration de poussière de respiration est situé, et établir les communications de données avec les capteurs de concentration de poussière de respiration ; et
   une étape de calibration : comparaison d'une valeur détectée sur site du dispositif individuel de détection de poussière de respiration avec une valeur détectée sur site du capteur de concentration de poussière de respiration, et calibration du capteur de concentration de poussière de respiration sur la base de la valeur détectée du dispositif individuel de détection de poussière de respiration.

5. Procédé de calcul de la quantité cumulée d'exposition à la poussière de la poussière de respiration de l'exploitant de la mine selon la revendication 1, dans lequel la trajectoire spatio-temporelle du personnel est obtenue sur la base de la position de l'exploitant signalée par un système de positionnement du personnel et le temps de séjour à la position, et dans lequel
   le système de positionnement du personnel comprend une pluralité de récepteurs de positionnement portés par l'exploitant et un point de dispositif pour recevoir les informations envoyées par les récepteurs de positionnement
   une relation topologique entre le point de dispositif et les récepteurs de positionnement est une structure arborescente, et les relations topologiques entre les récepteurs de positionnement comprennent des points et des segments d'arc, des segments d'arc et des segments d'arc, et des segments d'arc et des points.

**6.** Procédé de calcul de la quantité cumulée d'exposition à la poussière de la poussière de respiration de l'exploitant de la mine selon la revendication 1, dans lequel le procédé comprend en outre une étape consistant à effectuer une correction sur site sur le modèle de calcul pour la quantité individuelle d'exposition à la poussière de poussière de respiration, et dans lequel l'étape comprend :

utiliser un dispositif unique où de nombreux dispositif individuel de détection de poussière de respiration afin de détecter de manière continue à la position où le capteur de concentration de poussière de respiration est agencé sur la surface de fonctionnement pendant une période $t_1$, et corriger le modèle de calcul pour la quantité individuelle d'exposition à la poussière de poussière de respiration en utilisant une valeur détectée.

**7.** Procédé de calcul de la quantité cumulée d'exposition à la poussière de la poussière de respiration de l'exploitant de la mine selon la revendication 1, dans lequel le procédé comprend en outre une étape de toute optimisation du modèle de calcul pour la quantité individuelle d'exposition à la poussière de poussière de respiration, et dans lequel l'étape comprend :

respectivement transporter, par une pluralité d'exploitants, des dispositifs individuels de détection de poussière de respiration pour fonctionner pendant au moins un quart de travail sur la surface de fonctionnement, et optimiser le modèle de calcul pour la quantité individuelle d'exposition à la poussière de poussière de respiration en utilisant les valeurs détectées de la pluralité de dispositifs individuels de détection de poussière de respiration.

**8.** Système permettant de mesurer une quantité cumulée d'exposition à la poussière de poussière de respiration d'un exploitant minier en utilisant le procédé selon l'une quelconque des revendications 1 à 7, comprenant un centre de traitement de données, des capteurs de concentration de poussière de respiration, des capteurs de vitesse du vent, des capteurs d'humidité, un système de positionnement du personnel, une mémoire de données et un instrument de calibration, dans lequel les capteurs de concentration de poussière de respiration, les capteurs de vitesse du vent, les capteurs d'humidité et le système de positionnement du personnel sont en connexion sans fil avec le centre de traitement de données, et la mémoire de données est en connexion filaire avec le centre de traitement de données ; et dans lequel

la quantité de stockage de la mémoire de données comprend une trajectoire spatio-temporelle personnelle de tous les exploitants sur chaque surface de fonctionnement d'une mine et une loi de distribution de la concentration de poussière de respiration de la surface de fonctionnement,

le nombre de capteurs de concentration de poussière de respiration est pluriel, qui sont disposés de manière égale ou inégale dans une zone avec une diffusion de poussière relativement uniforme de la surface de fonctionnement, à des lieux de fonctionnement fixes et dans une plage de fonctionnement,

le nombre de capteurs de vitesse du vent est pluriel, qui sont disposés de manière égale ou inégale sur un côté au vent et un côté sous le vent de la surface de fonctionnement, au niveau d'un site d'interférence d'écoulement du vent et dans la plage de fonctionnement de l'exploitant,

le nombre de capteurs d'humidité est pluriel, qui sont disposés de manière égale ou inégale sur les surfaces de fonctionnement,

le système de positionnement du personnel comprend une pluralité de récepteurs de positionnement portés par l'exploitant et un point de dispositif pour recevoir les informations envoyées par les récepteurs de positionnement, une relation topologique entre le point de dispositif et les récepteurs de positionnement est une structure arborescente, et la relation topologique parmi les récepteurs de positionnement comprend des points et des segments d'arc, des segments d'arc et des segments d'arc, et des segments d'arc et des points,

l'instrument de calibration comprend un échantillonneur individuel de poussière de respiration et un dispositif de détection individuel de poussière de respiration ; et

le centre de traitement de données obtient la loi de distribution de la concentration de poussière de respiration et la trajectoire spatio-temporelle du personnel respectivement sur la base de données de sortie au moyen du capteur de concentration de poussière de respiration, des capteurs de vitesse du vent, du système de positionnement du personnel et des capteurs d'humidité, et combine le modèle de calcul pour la quantité individuelle d'exposition à la poussière de poussière de respiration pour calculer la quantité cumulée d'exposition à la poussière de poussière de respiration de l'exploitant.

Fig.1

Fig.2

| Individual respiration dust sampler | Standardizing | Individual respiration dust detection device | Calibrating | Respiration dust concentration sensor |
|---|---|---|---|---|

Fig.3

Fig.4

Fig.5

Sensor point 1

Sensor point 2

• • •

Sensor point n

Correction

Individual respiration dust detector

Calculation model for individual dust exposure amount of respiration dust

Fig.6

Operating surface

Individual respiration dust detector 1

Individual respiration dust detector 2

• • •

Individual respiration dust detector n

Optimization

Calculation model for individual dust exposure amount of respiration dust

Fig.7

**EP 3 557 222 B1**

REFERENCES CITED IN THE DESCRIPTION

**Patent documents cited in the description**

- CN 105089703 A